# EUROPEAN PATENT SPECIFICATION

(11) **EP 0 642 798 B1**
(45) Date of publication and mention of the grant of the patent: **18.04.2007**
(21) Application number: 93309720.6
(22) Date of filing: 03.12.1993
(51) Int. Cl.: A61K 48/00, A61K 47/48

(54) **Chemically-defined non-polymeric valency platform molecules and conjugates thereof**
Chemisch definierten nicht-polymer wertigen Plattformmolokülen und ihren Konjugaten
Molécules "platform" définis et de valence non-polymérique et leur conjugés

(30) Priority: 08.09.1993 US 118055; 22.10.1993 US 142598; 15.11.1993 US 152506; 22.11.1993 EP 93309288
(43) Date of publication of application: 15.03.1995
(60) Divisional application: 07004965.5
(73) Proprietor: LA JOLLA PHARMACEUTICAL COMPANY, San Diego, California 92121 (US)
(72) Inventor: Coutts, Stephen, Rancho Sante Fe, CA 92067 (US); Jones, David S., San Diego, CA 92127 (US); Livingston, Douglas Alan, San Diego, CA 92122 (US); Yu, Lin, San Diego, CA 92122 (US)
(74) Representative: Woods, Geoffrey Corlett

(56) References cited:
- EP-A- 0 442 724
- WO-A-87/00056
- WO-A-88/09810
- WO-A-95/07073
- US-A- 5 276 013
- US-A- 5 552 391
- US-A- 5 606 047
- US-A- 5 633 395
- DATABASE WPI Section Ch, Week 9130 Derwent Publications Ltd., London, GB; Class A96, AN 91-216937 XP002011738 & EP 0 438 259 A (LA JOLLA PHARMACEUTICAL COMPANY)
- JONES, DAVID S. ET AL: "Conjugates of Double-Stranded Oligonucleotides with Poly(ethylene glycol) and Keyhole Limpet Hemocyanin: A Model for Treating Systemic Lupus Erythematosus" BIOCONJUGATE CHEM., VOL. 5, NO. 5, PAGE(S) 390-9, 1994, XP000465950
- JONES, DAVID S. ET AL: "Immunospecific Reduction of Antioligonucleotide Antibody-Forming Cells with a Tetrakis-oligonucleotide Conjugate (LJP 394), a Therapeutic Candidate for the Treatment of Lupus Nephritis" JOURNAL OF MEDICINAL CHEMISTRY., vol. 38, no. 12, 1995, US, pages 2138-2144, XP002064253
- HERTLER AA: "HUMAN IMMUNE RESPONSE TO IMMUNOTOXINS" CANCER TREAT RES, 1988, VOL. 37, PAGE(S) 475-80, XP000600646
- SEHON A.H.: "Suppression of antibody responses by conjugates of antigens and monomethoxypoly(ethylene glycol)" ADV. DRUG DELIV. REV., 1991, VOL. 6, NO. 2, PAGE(S) 203-217, NL, XP000601424
- EFIMOV V A ET AL: "SYNTHESIS OF POLYETHYLENE GLYCOL - OLIGONUCLEOTIDE CONJUGATES" BIOORG KHIM, VOL. 19, NO. 8, PAGE(S) 800-804, August 1993, XP002013310

## Description

### Technical Field

This invention relates to conjugates comprising chemically-defined valency platform molecules coupled to polynucleotides for treating disease such as the autoimmune disease systemic lupus erythematosus (SLE or "lupus"). The invention also relates to the chemically-defined valency platform molecules.

### Background

A number of compounds have been employed as carriers for biologically useful molecules in preparing conjugates that are alleged to be tolerogenic. For example, Benacerraf, Katz, and their colleagues investigated and described the use of conjugates of the random co-polymer D-glutamic acid/D-lysine, referred to as D-GL in earlier literature (hereinafter D-EK) with haptens and various antigens to induce specific immune tolerance. See US Pats. Nos. 4,191,668 and 4,220,565.

Other investigators have studied conjugates of nucleosides or DNA with other carriers. Borel *et al.* (Science (1973) 182:76) evaluated the ability of isogenic mouse IgG-nucleoside conjugates to reduce the antibody response to denatured DNA in young animals of the NZB mouse strain. In separate studies Parker *et al.* (J. Immunol. (1974) 113:292) evaluated the effect of denatured DNA conjugate to poly-D-lysine and/or cyclophosphamide on the progression of the above-described syndrome in NZB mice.

In a later article (Ann NY Acad Sci (1986) 475:296-306) Borel *et al.* describe oligonucleotide-immunoglobulin conjugates. Borel *et al.* (J. Clin Invest (1988) 82:1901-1907 or US Patent No. 4,650,675) have described *in vitro* studies using conjugates of human immunoglobulin linked to DNA. US Patent No. 5,126,131 (Dintzis *et al.)* also relates to conjugates comprising carriers and molecules involved in immune responses.

Other references describe conjugates of nonimmunogenic polymers and immunogens (Saski *et al.,* Scand. J. Immun. (1982) 16:191-200; Sehon, Prog. Allergy (1982) 32:161-202; Wilkinson *et al.,* J. Immunol. (987) 139:326-331, and Borel *et al.,* J. Immunol. Methods (1990) 126:159-168).

In commonly-owned US Application Serial No. 07/914,869 (US Patent No. 5,276,013), US Patent No. 5,162,515 and US Application Serial No. 07/652,648 (US Patent No. 5,268,454) and in EP-A-0438259, conjugates comprising polymeric carriers such as D-EK, polyethylene glycol, poly-D-lysine, polyvinyl alcohol, polyvinyl pyrrolidone and immunoglobulins are described.

Efimov *et al,* Bioorganicheskaya Khimiya, vol. 19, no. 8, 800-804, 1993 discloses a method for the synthesis of 3'-, 5'- and 3', 5'-PEG-oligonucleotide conjugate molecules. WO 88/09810 discloses oligonucleotide conjugates where oligonucleotides are joined through a linking arm to a hydrophobic moiety. EP-A-0442724 discloses PEGylated interleukin-6 derivatives.

WO 87/00056 describes pharmaceutical compositions comprising a non-toxic inert pharmaceutically acceptable aqueous carrier medium in which is dissolved a conjugate of a protein selected from β-interferon, interleukin-2 and an immunotoxin wherein the protein is covalently conjugated to a water-soluble polymer selected from polyethylene glycol homopolymers and polyexyethylated polyols.

In sum, it is believed that the prior art shows only ill-defined chemical compounds or compounds with numerous non-specific attachment sites employed as valency platform molecules in conjugates. Because the valency of such compounds, the specific location of the attachment sites and the number of attachment sites are unpredictable and fluctuate widely, prior art conjugates comprising such compounds cannot be made reproducibly and show wide ranges in their reported activity.

### Disclosure of the Invention

In contrast to the above-described art, conjugates have now been developed which comprise chemically-defined valency platform molecules wherein the valency of the platform molecules is predetermined and wherein each attachment site is available for binding of a biological or chemical molecule. Valency platform molecules within the present invention are defined with respect to their chemical structure, valency, homogeneity and a defined chemistry which is amenable to effective conjugation with the polynucleotide.

Accordingly the present invention provides a conjugate comprising a polynucleotide coupled to a valency platform molecule, which conjugate has the general formula (I): 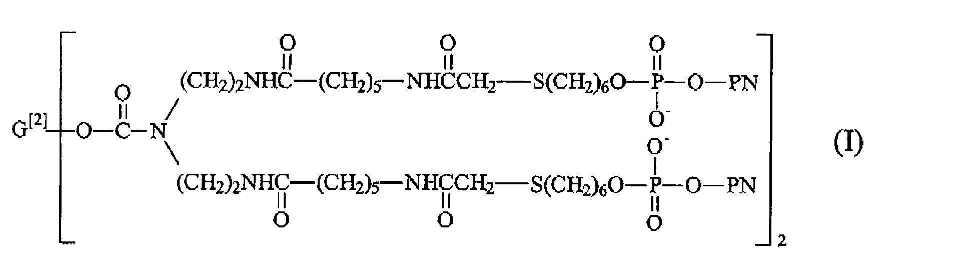 wherein G^{[2]} is -CH₂(CH₂OCH₂)ᵣCH₂- wherein r = 0 to 300 and PN is a polynucleotide.

The invention further provides:
- a method of making a conjugate of formula (I) wherein the polynucleotide is a polynucleotide duplex of at least 20 base pairs, which method comprises:
   (a) conjugating a polynucleotide-linker of general formula (II): 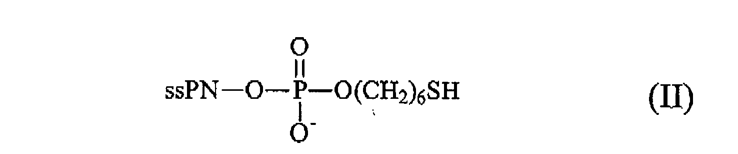 wherein ssPN denotes a single-stranded polynucleotide of at least 20 nucleotides in length, to a valency platform molecule of the general formula (III): 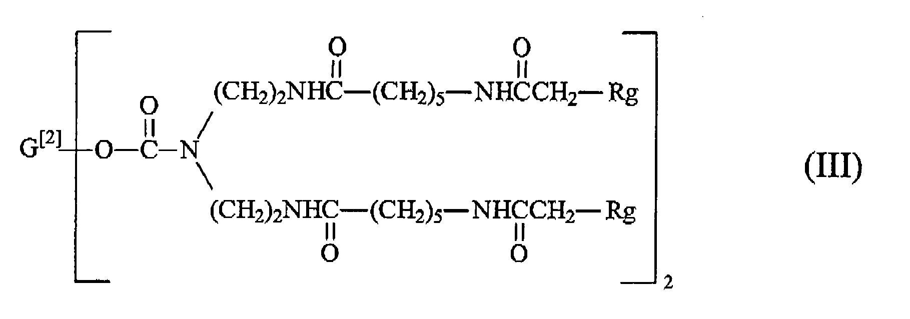 wherein Rg is halogen and G^{[2]} is as defined herein; and
   (b) annealing complementary single-stranded polynucleotides to each said single-stranded polynucleotide ssPN;
- a pharmaceutical composition comprising a conjugate of the invention and a pharmaceutically acceptable vehicle; and
- a valency platform molecule of the general formula (III): 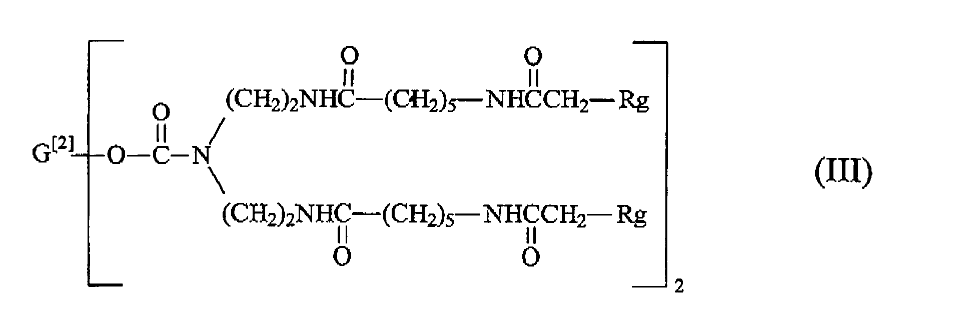
wherein G^{[2]} is -CH₂(CH₂OCH₂)ᵣCH₂- wherein r = 0 to 300 and Rg is halogen.

In a further preferred embodiment for treating lupus, the conjugate comprises the chemically-defined valency platform molecule and a multiplicity of polynucleotide duplexes of at least about 20 base pairs each bound to the platform molecule, and having significant binding activity for human SLE anti-dsDNA autoantibodies. In these preferred embodiments, the polynucleotide duplexes are substantially homogenous in length and one strand of the duplex is conjugated to the valency platform molecule either directly or via a linker molecule. Usually synthetic polynucleotides are coupled to a linker molecule before being coupled to a valency platform molecule. Usually the linker containing strand of the duplex is coupled at or proximate (i.e. within about 5 base pairs) one of its ends such that each strand forms a pendant chain of at least about 20 base pairs measured from the site of attachment of the strand to the linker molecule. The second strand is then annealed to the first strand to form a duplex.

A suitable linker molecule within the present invention is the 6 carbon thiol HAD, a thio-6 carbon chain phosphate. Chemically-defined valency platform molecules within the present invention are formed, for example, by reacting PEG-bis-chloroformate with N,N-di(2-[6'-N'-carbobenzyloxyaminohexanoamido]ethyl)amine (hereinafter "AHAB").

Surprisingly unexpected results of at least approximately ten fold up to more than one-hundred fold increase in immunosuppression are achieved using conjugates comprising the chemically-defined valency platform molecules of the instant invention and a polynucleotide when compared to the polymeric carriers described in the prior art. For example, at least a one hundred-fold increase in the immunosuppression of anti-dsDNA autoantibodies was achieved as described herein using conjugates within the present invention comprising chemically-defined valency platform molecules when compared to conjugates comprising an ill-defined carrier described in the prior art.

Still another aspect of the invention is a conjugate of (a) the chemically-defined valency platform molecule and (b) a multiplicity of polynucleotide duplexes each and all of which is bound to the valency platform by a functional group located at or proximate a terminus of one of the strands of the duplex, said conjugate being a human SLE tolerogen.

The conjugates of the invention can thus be used in a method of treatment of the human or animal body by therapy, An individual can thus be treated for SLE by administering to the individual an effective amount of the above-described conjugates.

The conjugates of the invention are made by a method comprising: covalently bonding a polynucleotide to a chemically-defined valency platform molecule to form a conjugate. Thus, the conjugates for treating SLE described above can be made by a method comprising: reacting a multiplicity of single-stranded polynucleotides each of which is at least about 20 nucleotides in length and has a functional group at or proximate one of its termini that reacts with functional groups on the chemically-defined valency platform molecule to form a conjugate, and annealing complementary single-stranded polynucleotides to the single-stranded polynucleotides conjugated to the chemically-defined valency platform molecule to form pendant chains of double-stranded DNA.

### Brief Description of the Drawings

Figure 1 shows the anti-PN response in mice primed with PN-KLH, treated with (PN)₂₀-HAD-AHAB-TEG, Conjugate 20-II, in the doses shown or with HAD-ARAB only, or the PN only or a mixture of each, then boosted with PN-KLH and bled 5 days later. Sera were tested by the Farr assay using radiolabeled PN at a concentration of 10⁻⁸ M. The percent reduction was calculated and the data are presented. There were 5 mice per group.

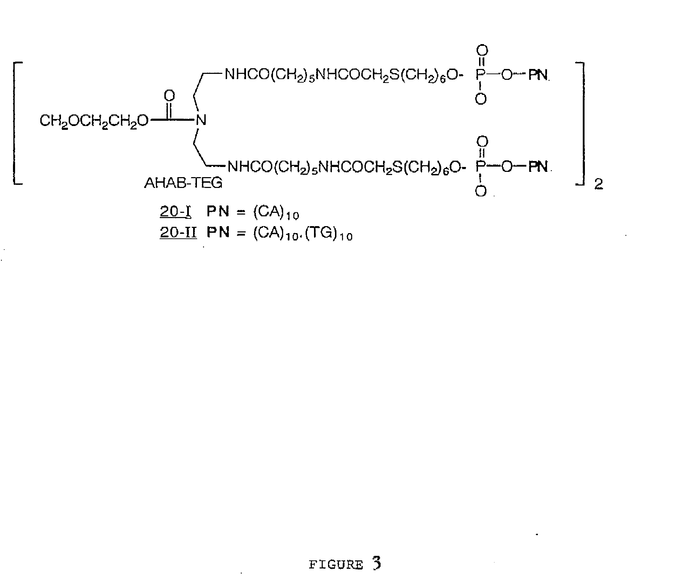

Figures 2 and 3 show the structure of the derivatized valency platform molecule and the linker coupling the polynucleotide to the platform molecule for Conjugates 3-I and 3-II (Figure 2) and 20-I and 20-II (Figure 3).
Figure 4 shows the structures of the derivatized valency platform molecule "HAD-AHAB-TEG".
Figure 5 illustrates the increase in the percentage of reduction in anti-dsPN antibody achieved by conjugates LJP-249A and LJP-249B, which are Conjugate 3-II, compared to a prior art conjugate (LJF-105) comprising D-EK and (PN)₅₀.
Figure 6 illustrates the suppression of serum circulating IgG anti-DNA antibodies in male BXSB mice treated with LJP-394, Conjugate 20-II. An ELISA assay was used to measure IgG antibodies to (PN)₅₀ conjugated to D-EK. The serum from each of eight individual mice in each group was assayed.

### Modes for Carrying out the Invention

As used herein "valency platform molecule" means a chemically-defined nonimmunogenic molecule containing sites which facilitate the attachment of a discreet number of biological and/or chemical molecules.

"Nonimmunogenic" is used to describe the valency platform molecule and means that the valency platform molecule elicits substantially no immune response when it is administered by itself to an individual.

As used herein "individual" denotes a member of the mammalian species and includes humans, primates, mice and domestic animals such as cattle and sheep, sports animals such as horses, and pets such as dogs and cats.

The valency of a chemically-defined valency platform molecule within the present invention is predetermined by the number of branching groups added to the platform molecule. A conjugate within the instant invention is biologically stabilized; that is, it exhibits an *in vivo* excretion half-life of hours to days to months to confer therapeutic efficacy. The chemically-defined valency platform molecules of the instant invention are also substantially nonimmunogenic (i.e., they exhibit no or only mild immunogenicity when administered to animals), non-toxic at the doses given (i.e., they are sufficiently non-toxic to be useful as therapeutic agents) and are preferably composed of a defined chemical structure. They provide a non-immunogenic, non-toxic polyfunctional substrate to which polynucleotide duplexes may be attached covalently. They will normally have an average molecular weight in the range of about 200 to about 200,000, usually about 200 to about 20,000, and are homogeneous as compared to the prior art polymers which were a mixture of compounds of widely fluctuating molecular weight. Examples of particularly preferred, homogenous valency platform molecules within the present invention are derivatized triethylene glycol (TEG) and polyethylene glycols (PEGs) having a molecular weight of about 200 to about 8,000.

The synthetic polynucleotide duplexes that are coupled to the valency platform molecule are composed of at least 20 bp and preferably 20-50 bp. Polynucleotides described herein are deoxyribonucleotides unless otherwise indicated and are set forth in 5' to 3' orientation. Preferably the duplexes are substantially homogeneous in length; that is, the variation in length in the population will not normally exceed ±20%, preferably ±10%, of the average duplex length in base pairs. They are also preferably substantially homogeneous in nucleotide composition; that is, their base composition and sequence will not vary from duplex to duplex more than 10%. Most preferably they are entirely homogeneous in nucleotide composition from duplex to duplex.

Based on circular dichroic (CD) spectra interpretation, the duplexes that are useful in the invention assume a B-DNA type helical structure. It should be understood that it is not intended that the invention be limited by this belief and that the duplexes may, upon more conclusive analysis assume Z-DNA and/or A-DNA type helical structures.

These polynucleotide duplexes may be synthesized from native DNA or synthesized by chemical or recombinant techniques. Naturally occurring or recombinantly produced dsDNA of longer length may be digested (e.g., enzymatically, chemically or by mechanical shearing) and fractionated (e.g., by agarose gel or Sephadex® column) to obtain polynucleotides of the desired length.

Alternatively, pairs of complementary single-stranded polynucleotide chains up to 70 bases in length are readily prepared using commercially available DNA synthesizers and then annealed to form duplexes by conventional procedures. Synthetic dsDNA of longer length may be obtained by enzymatic extension (5'-phosphorylation followed by ligation) of the chemically produced shorter chains.

The polynucleotides may also be made by molecular cloning. For instance, polynucleotides of desired length and sequence are synthesized as above. These polynucleotides may be designed to have appropriate termini for ligation into specific restriction sites. Multiple iterations of these oligomers may be ligated in tandem to provide for multicopy replication. The resulting construct is inserted into a standard cloning vector and the vector is introduced into a suitable microorganism/cell by transformation. Transformants are identified by standard markers and are grown under conditions that favor DNA replication. The polynucleotides may be isolated from the other DNA of the cell/microorganism by treatment with restriction enzymes and conventional size fractionation (e.g., agarose gel, Sephadex® column).

Alternatively, the polynucleotides may be replicated by the polymerase chain reaction (PCR) technology. Saiki, R.K, et al., Science (1985) 230:1350; Sacki, et al., Science (1988) 239:487; Sambrook, et al., In Molecular Cloning Techniques: A Laboratory Manual, Vol. 12, p 14.1-14.35 Cold Spring Harbor Press (1989).

Polynucleotides may be screened for binding activity with SLE antisera by the assays described in the examples. The modified Farr assay in which binding activity may be expressed as I₅₀ (the polynucleotide concentration in molar nucleotides resulting in half-maximal inhibition) is a preferred assay. Polynucleotide duplexes having an I₅₀ of less than about 500 nM, preferably less than 50 nM, are deemed to have significant binding activity and are, therefore, useful for making the conjugates of this invention.

The polynucleotides are conjugated to the chemically-defined valency platform molecule in a manner that preserves their antibody binding activity. This is done by conjugating the polynucleotide to the valency platform molecule at a predetermined site on the polynucleotide chain such that the polynucleotide forms a pendant chain of at least 20 base pairs measured from the conjugating site to the free (unattached) end of the chain.

In a particularly preferred embodiment, the polynucleotides of the invention conjugates are coupled to a linker molecule at or proximate one of their ends. The linker molecule is then coupled to the chemically-defined valency platform molecule. For example, a defined double-stranded PN can be conjugated to a valency platform molecule by first providing a single chain consisting of 20 alternating cytosine (C) and adenosine (A) nucleotides. Four CA chains can then be covalently conjugated through linkers such as HAD to four reactive sites on a derivatized platform molecule such as triethylene glycol. The valency platform molecule is synthesized to include groups such as bromoacetyl. During the conjugation, a leaving group is displaced by sulfur. A second single nucleotide chain consisting of 20 alternating thymidine (T) and guanosine (G) nucleotides can then be annealed to the CA strand to form a double-stranded PN conjugate of the formula, [(PN)₂₀-linker]₄-valency platform molecule.

The conjugates will normally be formulated for administration by injection, (e.g., intraperitoneally, intramuscularly, intravenously etc.). Accordingly, they will typically be combined with pharmaceutically acceptable aqueous carriers such as saline, Ringer's solution, dextrose solution, and the like. The conjugate will normally constitute about 0.01% to 10% by weight of the formulation. The conjugate is administered to an individual in amounts sufficient to at least partially reestablish tolerance to the autoantigens causing SLE. Such amounts are sometimes herein referred to as "therapeutically effective" amounts. The particular dosage regimen i.e., dose, timing and repetition, will depend upon the particular individual, and that individual's medical history. Normally a dose of about 1 to 1000 µg conjugate/kg body weight will be given. Repetitive administrations may be required to achieve and/or maintain a state of immune tolerance.

The following examples further illustrate the invention and its unexpectedness relative to the prior art.

### Example 1

The following reaction schemes illustrate methods of synthesizing derivatized chemically-defined valency platform molecules within the present invention. In this example, DMTr=4,4'-dimethoxytriphenylmethyl; Tr=trityl; Bz=benzoyl; Cp=deoxycytidine monophosphate, CE=cyanoethyl; CPG=controlled pore glass, DMF = dimethyl formamide, DCC = dicyclohexylcarbodiimide, TFA = trifluoroacetic acid, CDI = carbonyl diimidazole, Ts = tosyl (para-toluene sulfonyl), DIPAT = diisopropyl ammonium tetraazolide, TBDMSCl = tertbutyl dimethyl silyl chloride, TBAF = tetrabutyl ammonium fluoride, IA-DABA = 3,5-bis-(iodoacetamide)-benzoyl.

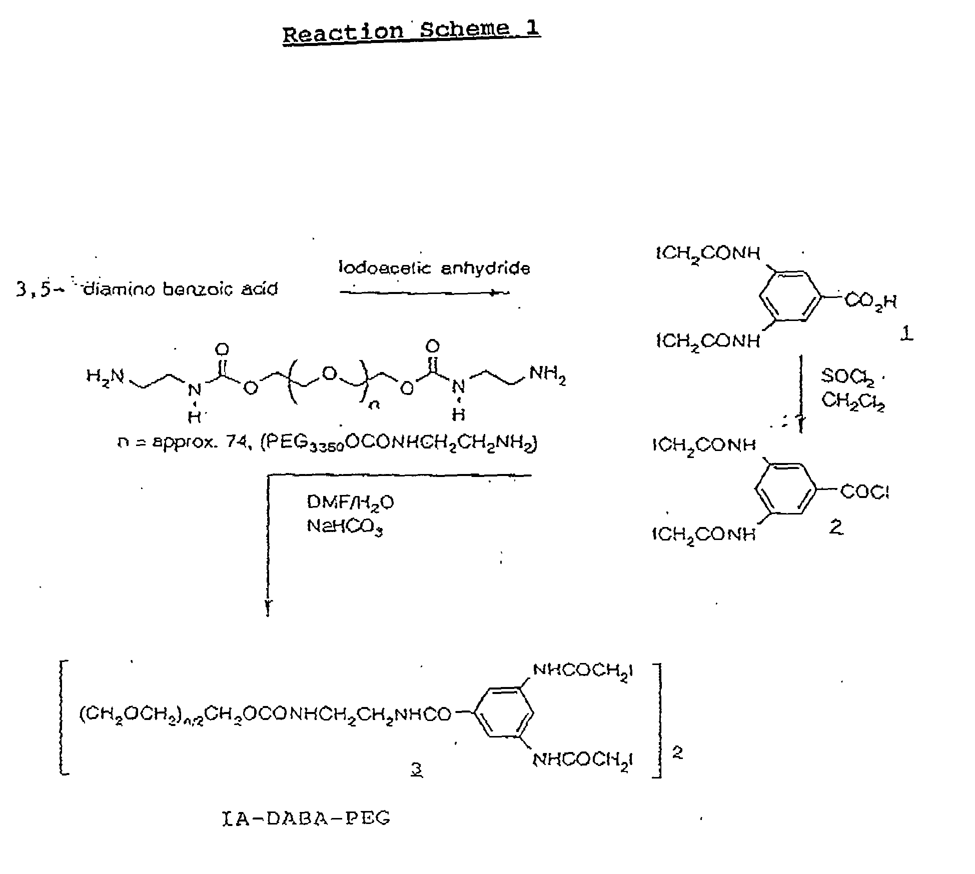

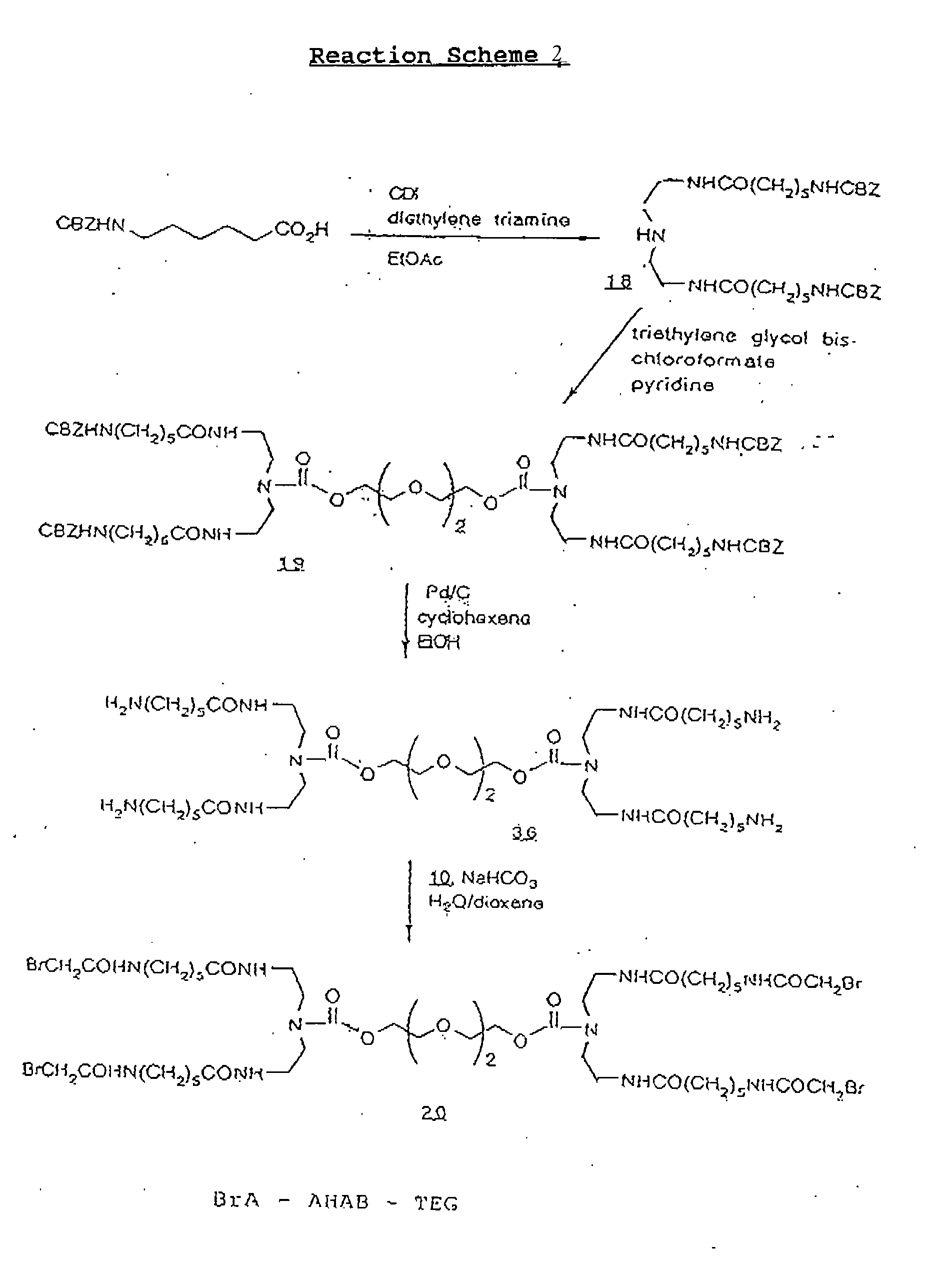

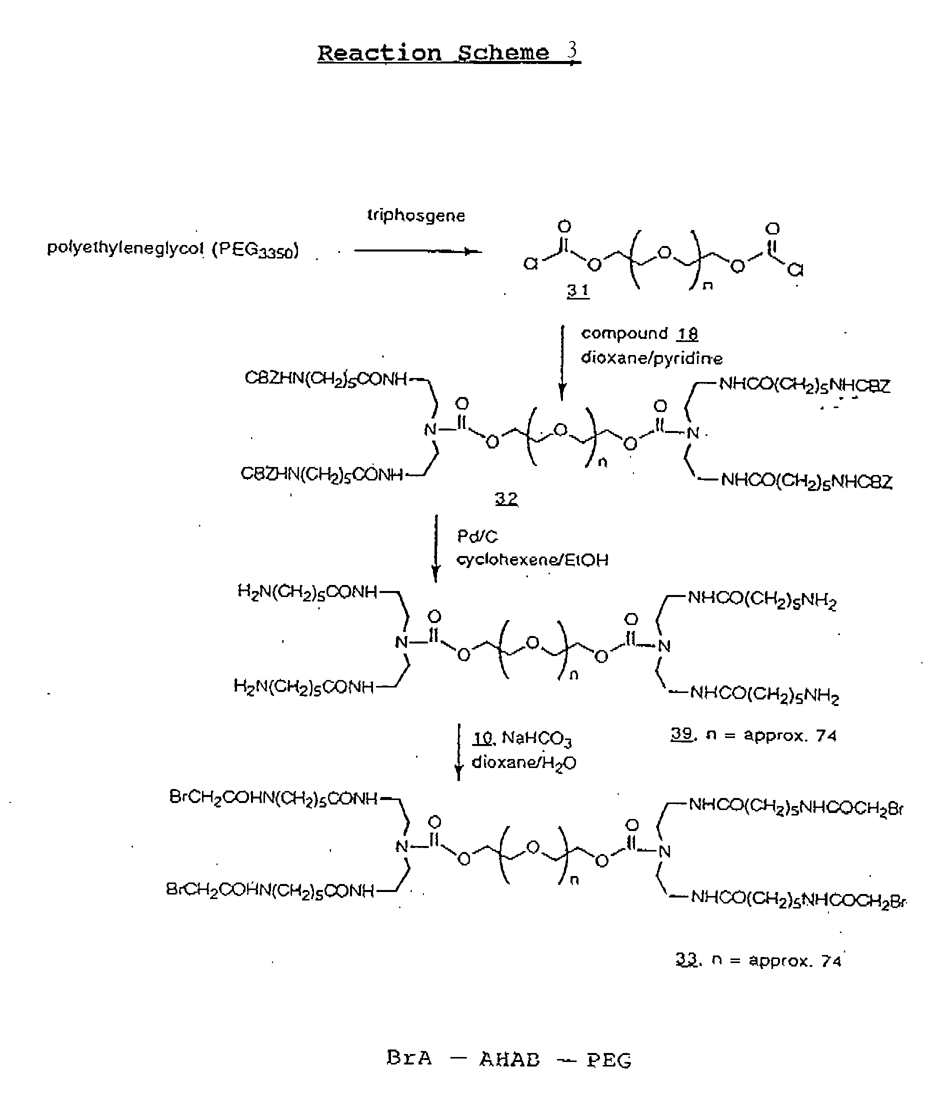

synthesis of reagents used to modify (CA)₈,(CA)₁₀, (CA)₁₂ and (CA)₁₆ with disulfide linkers is described in Reaction Scheme 4 below:

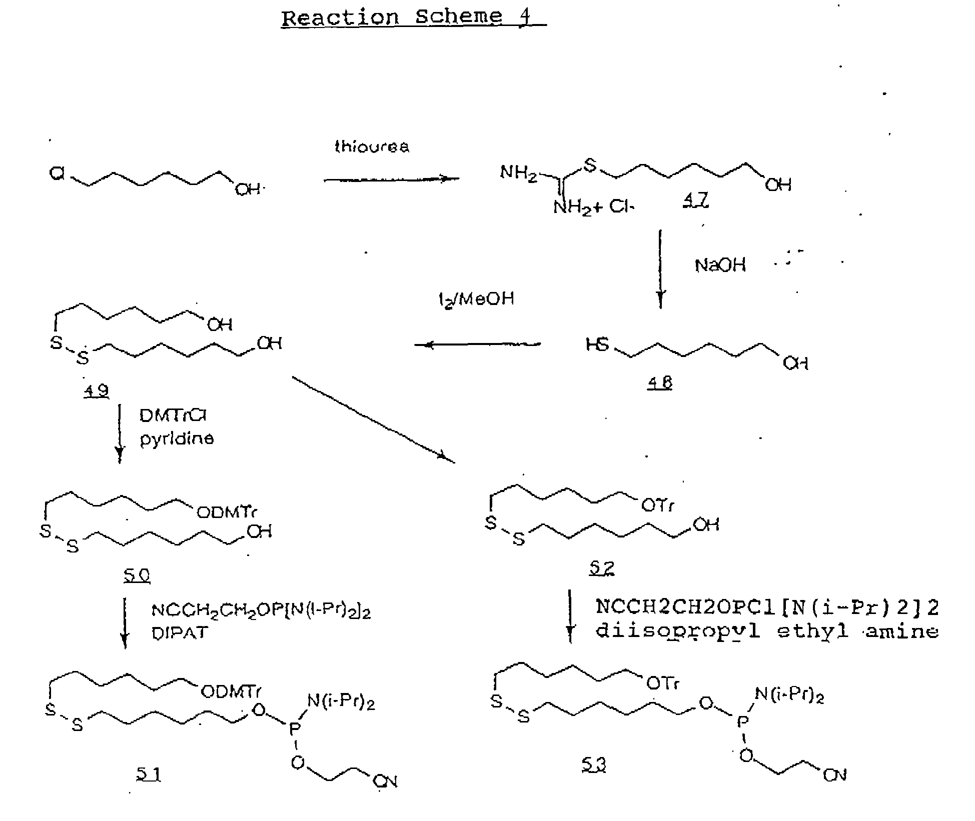

Synthesis of a reagent used to modify (CA)₂₅ with vicinal diol linkers is described in Reaction Scheme 5 below:

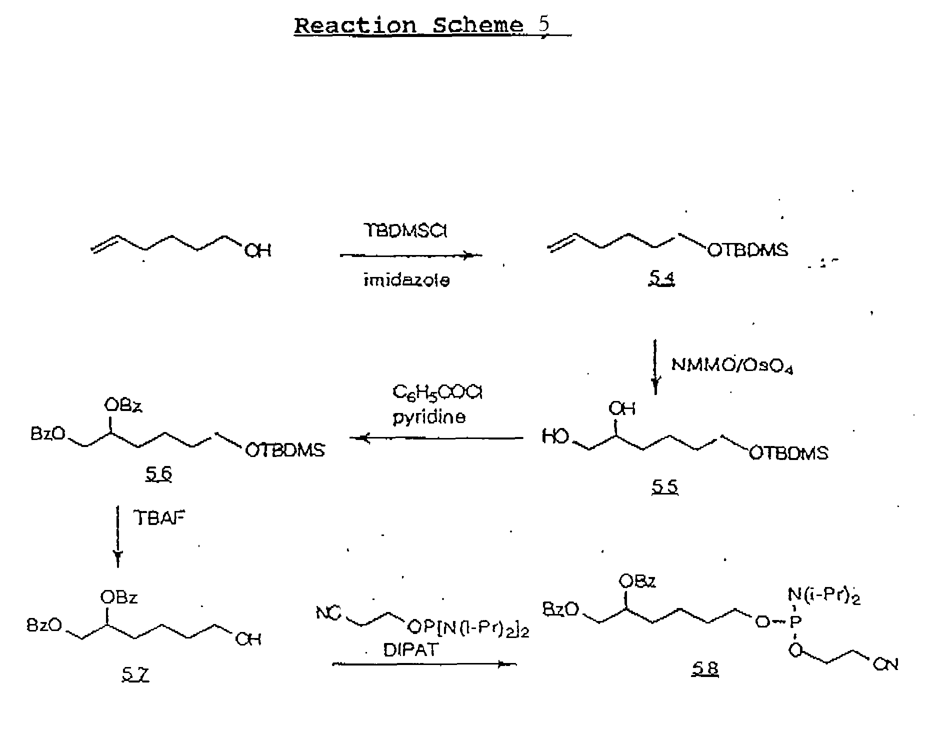

### Example 2

### Synthesis of Chemically-Defined Valency Platform Molecules

Reference Compound 1 - [3,5-Bis-(iodoacetamido)benzoic acid]: 2.93 g (8.28 mmol, 2.2 eq) of iodoacetic anhydride was added to a stirred suspension of 572 mg (3.76 mmol) of 3,5-diaminobenzoic acid in 19 mL of dioxane at room temperature under N₂ atmosphere. The mixture was stirred, covered with foil for 20 hours and partitioned between 50 mL of EtOAc and 50 mL of 1N HCl solution. The EtOAc layer was washed with brine, dried over MgSO₄, filtered, and concentrated on a rotary evaporator to give 3.3 g of tan solid. The material was purified by silica gel chromatography (94/5/1 CH₂Cl₂/MeOH/HOAc) to yield 992 mg (54%) of compound 1 as a white solid: NMR (DMSO) 3.84 (s, 4H), 7.91 (s, 2H), 8.14 (s, 1H), 10.56 (s, 2H).

Reference Compound 2 - [3,5-Bis-(iodoacetamido)benzoyl chloride]: 117 µL (1.6 mmol, 190 mg) of SOCl₂ was added to a solution of 390 mg (0.8 mmol) of 1 in 34 mL of THF. The mixture was refluxed under N₂ atmosphere until all solids had dissolved (approximately 30 minutes) to give a clear red-brown solution. The mixture was concentrated on the rotary evaporator and placed under vacuum to provide crude compound 2 as a foamy solid which was used directly in the next step.

Reference Compound 3 - [N,N'-Bis-(3,5-Bis-(iodoacetamido)benzoyl) derivative of α,ω-bis-(N-2-aminoethylcarbamoyl)polyethyleneglycol]: 570 mg of α,ω-bis-(N-2-aminoethylcarbamoyl)polyethyleneglycol (0.16 mmol, 3350 g/mol, Sigma) was placed in a tared flask.

Toluene (20 mL) was added and water was removed by azeotropic distillation. The residue was dried under vacuum to give 549 mg of solid and dissolved in 4 mL THF with 89 *µ*L (0.64 mmol) of diisopropylethylamine. The crude acid chloride was dissolved in 4 mL anhydrous THF and added to the mixture over 30 seconds under N₂. The mixture was stirred for 16 hours at room temperature and partitioned between 25 mL of 0.1 N HCl and 25 mL of CH₂Cl₂. The aqueous layer was again extracted with CH₂Cl₂ and the organic layers were combined, washed with 25 mL of H₂O, followed by 50 mL, of at NaHCO₃ solution. The organic layers were dried with Na₂SO₄, filtered, and concentrated to give 784 mg of orange oil. Silica gel chromatography (9/1 CH₂Cl₂/MeOH) yielded 190 mg of colorless oil which was crystallized from hot EtOH/Et₂O, collected on sintered glass filter under N₂ pressure, and dried under vacuum to provide 177 mg of compound 3 as a white solid: NMR (CDCl₃) 3.40 (bd m, 8H), 3.59 (bd s, (CH₂CH₂O)ₙ, integral too large to integrate in relation to other integrals), 3.91 (s, 8H), 4.21 (m, 4H), 6.04 (bd m, 2H), 7.55 (bd m, 2H), 7.78 (bd s, 4H), 8.10 (bd s, 2H), 9.30 (bd m, 4H): iodoacetyl determination (European Journal of Biochemistry (1984) 140:63-71): Calculated, 0.92 mmol/g; Found, 0.96 mmol/g.

Compound 10 - [4-Nitrophenylbromoactate]: 9.28 g (45 mmol) of dicyclohexylcarbodiimide was added to a stirred solution of 5.0 g (35.9 mmol) of bromoacetic acid and 8.50 g (61.1 mmol) of 4-nitrophenol in 180 mL of EtOAc at 0°. The mixture was stirred for 16 hours at 5° and 1 mL of acetic acid was added. The mixture was stirred for 20 minutes at room temperature and then placed in a freezer for 20 minutes. The solid material was removed by filtration, and the filtrate was concentrated to a viscous oil and crystallized from Et₂O/hexanes to provide 7.73 g (83%) of compound 10 as flakes: m.p. 86-87°; TLC Rf = 0.63 (50/50/1 hexanes/EtOAc/HOAc); ¹H NMR (CDCl₃) d 4.13 (s, 2H), 7.36 (d, J=12 Hz, 2H), 8.32 (d, J=12 Hz, 2H); ¹³C NMR (CDCl₃) d 24.9, 122.1, 125.3, 155.5 164.9; Anal. calc'd for C₈H₆BrNO₄: C, 36.95; H, 2.33; N, 5.39. Found: C, 37.24; H, 2.33; N, 5.42.

Compound 18 - [1,5-Bis(N-carbobenzyloxy-6-aminohexanoamido)-3-azapentanel: 3.09 g (19.0 mmol) of carbonyldiimidazole was added to a solution of 5.05 g (19.0 mmol) of N-carbobenzyloxy-6-aminohexanoic acid in 25 mL of EtOAc at room temperature. The mixture was stirred for 15 hours and 1.02 mL (982 mg, 9.52 mmol) of diethylenetriamine was then added followed by 2.65 mL (1.93 g, 19.0 mmol) of Et₃N. The resulting mixture was stirred for 4 hours, and the solid product was collected by filtration. Recrystallization (MeOH/EtOAc) gave 4.27 g (75%) of compound 18 as a fine grainy solid: m.p. 132-133°; ¹H NMR (CDCl₃) d 1.33 (m, 4H), 1.52 (m, 4H), 1.64 (m, 4H), 2.18 (t, 4H), 2.73 (t, 4H), 3.16 (m, 4H), 3.35 (m, 4H), 4.96 (bd s, 2H), 5.09 (s, 4H), 6.13 (bd s, 2H), 7.33 (s, 10H); Anal. calc'd for C₃₂H₄₇N₅O₆: C, 64.29; H, 7.50; N, 11.72. Found: C, 63.54; H, 7.75; N, 11.91.

Compound 19: 657 µL (880 mg, 3.2 mmol) of triethyleneglycol-bis-chloroformate (Aldrich) was added to a solution of 4.86 g (8.1 mmol) of compound 18 in 162 mL of pyridine in a 20° water bath. The mixture immediately formed a precipitate. The mixture was stirred for 16 hours and the resulting cloudy yellow solution was concentrated under vacuum. The concentrate was partitioned between 150 mL of EtOAc and two 150 mL portions of 1 N HCl (making sure the aqueous layer was acidic). The aqueous layers were combined and extracted with a second 150 mL portion of EtOAc. The EtOAc layers were combined, dried (MgSO₄), filtered, and concentrated. The resulting residue was crystallized (EtOAc/hexanes/CHCl₃) to provide 1.92 g (43%) of compound 19 as fine yellow tinted crystals: m.p. 86-91°; ¹H NMR (CDCl₃) 1.31 (m, 8H), 1.52 (m, 8H), 1.62 (m, 8H), 2.20 (m, 8H), 3.20 (m, 8H), 3.39 (s, 16H), 3.62 (s, 4H), 3.68 (m, 4H), 4.26 (m, 4H), 5.08 (s, 8H), 5.32 (bd s, 4H), 7.31 (bd s, 4H), 7.37 (s, 20H); ¹³C NMR (CDCl₃) d 25.1, 26.2, 26.4, 29.6, 36.0, 36.2, 38.5, 38.8, 40.8, 64.5, 66.4, 69.1, 70.3, 128.0, 128.4, 136.7, 156.5, 156.9, 173.6; Anal. calc'd for C₇₂H₁₀₄N₁₀O₁₈: C, 61.87; H, 7.50; N, 10.02. Found: C, 61.68; H, 7.63; N, 9.95.

Compound 36: 3.5 mL of cyclohexene was added to a solution of 800 mg (0.57 mmol) of compound 19 in 5 mL of absolute EtOH. The solution was placed under nitrogen, 500 mg of 10% Pd on carbon was added, and the resulting mixture was refluxed with stirring for 2 hours. When cool, the mixture was filtered through diatomaceous earth and concentrated to give 500 mg (100%) of compound 36 as an oil: ¹H NMR (50/50 CDCl₃/CD₃OD) d 1.21 (m, 8H), 1.49 (m, 8H), 1.62 (m, 8H), 2.19 (t, J = 7.4 Hz, 8H), 2.67 (t, J = 7.4 Hz, 8H), 3.36 (bd s, 16H), 3.67 (s, 4H), 3.71 (m, 4H), 4.21 (m, 4H).

Compound 20: 3.9 g (46.4 mmol) of NaHCO₃ was added to a solution of 5.0 g (5.8 mmol) of compound 36 in 37.5 mL of dioxane and 12.5 mL of H₂O. The mixture was cooled to 0° in an ice bath and 8.7 g (34.8 mmol) of 4-nitrophenylbromoacetate, compound 10, was added. The mixture was stirred at 0° for 1 hour and 50 mL of 1 N H₂SO₄ was slowly added. The mixture was extracted with three, 50 mL portions of EtOAc. The EtOAc extracts were discarded and the aqueous layer was extracted with six, 50 mL portions of 20/80 MeOH/CH₂Cl₂. The combined MeOH/CH₇Cl₂ layers were dried (Na₂SO₄), filtered, and concentrated. The residue was purified by silica gel chromatography (step gradient 9/1 CH₂Cl₂/MeOH then 85/15/5 CH₂Cl₂/MeOH/THF) to provide 3.62 g (46%) of compound 20 as a white solid: melting point 66.0-70.5°. An analytical sample was prepared by preparative HPLC (C₁₈ reversed phase column, gradient 25/75/0.1 to 35/65/0.1 CH₃CN/H₂O/CF₃CO₂H over 50 minutes, 225 nm) to give a clear oil which solidified on standing under vacuum to give a white solid: melting point 87-89°; ¹H NMR (CDCl₃) d 1.35 (m, 8H), 1.55 (m, 8H), 1.64 (m, 8H), 2.26 (m, 8H), 3.28 (m, 8H), 3.42 (bd s, 16H), 3.66 (s, 4H), 3.70 (m, 4H), 3.89 (s, 8H), 4.19 (m, 4H); ¹³C NMR (CDCl₃) d 25.1, 26.2, 28.8, 29.0, 38.5, 39.1, 40.0, 47.8, 48.3, 64.7, 69.1, 70.3, 157.0, 166.3, 174.9; MS (FAB) m/e (relative intensity) MH+ [1341(25), 1343(60), 1345(70), 1347(56), 1349(21)], 705.6(100); Anal. calc'd for C₄₈H₈₄N₁₀O₁₄Br₄: C, 42.86; H, 6.29; N, 9.27; Br, 23.77. Found: C, 42.15; H, 6.28; N, 9.87; Br, 25.33.

Compound 31 - [PEG₃₃₅₀-bis-chloroformate]: Two drops of dry pyridine, followed by 125 mg (0.418 mmol) of triphosgene, was added to a solution of 1.0 gram (0.249 mmol) of polyethylene glycol (J.T. Baker, average molecular weight 3350 g per mol) which had been dried by azeotropic distillation (toluene) in 12 mL of CH₂Cl₂. The mixture was stirred at room temperature for 20 hours and the solvent was evaporated under vacuum to give 1.0 g (100%) of compound 31 as a white solid: ¹H NMR (CDCl₃) d 3.40-3.65 (m, approx. 300H, integral too large to be accurate), 3.77 (m, 4H), 4.46 (m, 4H).

Compound 32: A solution of 1.0 g (0.25 mmol) of compound 31 in 12 mL of 5:1 CH₂Cl₂/dioxane was added dropwise to a 50° solution of 600 mg (1.0 mmol) of compound 18 in 10 mL of dioxane and 1.5 mL of pyridine. The resulting cloudy solution was stirred for 72 hours. 25 mL of CH₂Cl₂ was added and the mixture was then filtered. The filtrate was evaporated and the semi-solid residue was purified by chromatography on G-10 Sephadex®. The resulting solid was crystallized (CH₂Cl₂/Et₂O) to give 829 mg (75%) of compound 32 as a faintly yellow solid: ¹H NMR (CDCl₃) d 1.30 (m, 8H), 1.40 (m, 8H), 1.61 (m, 8H), 2.18 (m, 8H), 3.17 (m, 8H), 3.40 (m, 16H), 3.62 (m, approx. 300H, integral too large to be accurate), 4.15 (m, 4H), 5.07 (s, 8H), 7.33 (m, 20H).

Compound 39: 100 mg of 10% Pd/C was added to a solution of 300 mg (0.065 mmol) of compound 32 in 5 mL of absolute EtOH and 2 mL of cyclohexene under nitrogen. This mixture was refluxed under nitrogen for 2 hours. The mixture was filtered through diatomaceous earth and the solvent was evaporated to give 237 mg (90%) of compound 39 as a white solid: ¹H NMR (CDCl₃) d 1.37 (m, 8H), 1.48 (m, 8H), 1.65 (m, 8H), 2.21 (m, 8H), 2.50 (m, 8H), 3.39 (m, 16H), 3.64 (m, approx. 300H, integral too large to be accurate), 4.19 (m, 4H).

Compound 33: 125 mg (0.67 mmol) of NaHCO₃ and 115 mg (0.44 mmol) of compound 10 was added to a solution of 225 mg (0.055 mmol) of 39 in 5 mL of dioxane and 5 mL of water. The resulting yellow solution was stirred at room temperature for 12 hours. The solution was then extracted with three 30 mL portions of CH₂Cl₂. The aqueous layer was acidified with 1 N H₂SO₄ and extracted with three, 30 mL portions of CH₂Cl₂. The combined CH₂Cl₂ layers were dried (MgSO₄), filtered, and concentrated to provide a yellow oil. Purification by chromatography on G-10 Sephadex® (MeOH) and recrystallization of the resulting oil (EtOH/Et₂O) provided 182 mg (73%) of compound 33 as a white solid: ¹H NMR (CDCl₃) d 1.35 (m, 8H), 1.55 (m, 8H), 1.65 (m, 8H), 2.22 (m, 8H), 3.28 (m, 8H), 3.42 (m, 16H), 3.50-364 (m, approx. 300H, integral too large to be accurate), 3.87 (s, 8H), 4.18 (m, 4H); bromoacetyl determination (European Journal of Biochemistry 1984, 140, 63-71): Calculated, 0.87 mmol/g; Found, 0.73 mmol/g. Anal Calcd. for C₁₉₁H₃₇₅O₈₇N₁₀Br₄: C, 50.84; H, 8.33; N, 3.09; Br, 7.05. Found: C, 51.98; H, 8.34; N, 2.45; Br, 10.19.

compound 47 - S-(6-hydroxyhexyl)isothiuronium chloride: 11.1 g (146 mmol) of thiourea was added to a solution of 16.6 mL (20.0 g, 146 mmol) of 6-chlorohexanol in 49 mL of ethanol and the mixture was refluxed for 24 hours. The mixture was cooled to 0° and the product crystallized. The crystals were collected by vacuum filtration and dried to give 28.4 g (92%) of compound 47 as a white solid: mp 122-124°; ¹H-NMR (DMSO) 1.40 (m, 4H), 1.65 (m, 2H), 3.21 (t, 2H), 3.41 (t, 2H), 9.27 and 9.33 (overlapping broad singlets, 4H); Anal. Calc'd for C₇H₁₇ClN₂OS: C, 39.51; H, 8.06; N, 13.17; S, 15.07. Found: C, 39.69; H, 8.00; N, 13.01; S, 15.16.

Compound 48 - 6-Mercaptohexan-1-ol: 9.25 g of NaOH pellets was added to a solution of 17.8 mg (83.6 mmol) of compound 47 in 120 mL of H₂O and 120 mL of EtOH. The mixture was refluxed for 4 hours. The mixture was carefully concentrated to approximately 75 mL and the concentrate was purified by vacuum distillation to provide 7.4 g (66%) of compound 48: bp 95-105° @ 5 mm Hg; ¹H NMR (CDCl₃) 1.41 (m, 9H), 2.59 (dt, 2H), 3.69 (t with underlying brd s, 3H); ¹³C NMR (CDCl₃) d 24.5, 25.2, 28.0, 32.5, 33.9, 62.7; Anal. calc'd for C₆H₁₄OS: C, 53.68, H, 10.51; S, 23.89. Found: C, 53.35; H, 10.72; S, 23.60.

Compound 49 - Bis-(6-hydroxyhexyl)disulfide: A solution of 4.02 g (15.8 mmol) of I₂ in 90 mL of MeOH was added dropwise over a period of 10 minutes to a solution of 4.26 g (31.7 mmol) of compound 48 in 10 mL of MeOH and 13.7 mL (9.97 g, 98.5 mmol) of Et₃N under N₂ atmosphere and cooled in an ice bath. The cooling bath was removed and the mixture was stirred at ambient temperature for 4 hours. The mixture was concentrated on the rotary evaporator and purified by silica gel chromatography (1:1 hexane/EtOAc) to provide 3.12 g (73%) of compound 49 as a pale yellow solid: TLC R_{f} .18 (1:1 hexane/EtOAc); mp 38-48°; ¹H NMR (CDCl₃) 1.15-2.20 (m, 16H), 2.73 (t, 4H), 3.70 (t, 4H); Anal. calc'd for C₁₂H₂₆S₂O₂: C, 54.09; H, 9.84; S, 24.06. Found: C, 54.85, H, 9.86; S, 24.11.

Compound 50 - Mono-O-(4',4"-dimethoxytriphenylmethyl)-bis-(6-hydroxyhexyl)disulfide: 3.97 g (11.7 mmol) of 4,4'-dimethoxytriphenylmethyl chloride was added to a solution of 3.12 g (11.7 mmol) of compound 49 and 45 mL of pyridine. The mixture was stirred at ambient temperature for 16 hours. Most of the pyridine was removed on the rotary evaporator and the residue was partitioned between 100 mL of saturated NaHCO₃ solution and 100 mL of EtOAc. The EtOAc layer was washed with 50 mL of saturated NaCl solution, dried (Na₂SO₄), filtered and concentrated to an oil. Purification by silica gel chromatography (9:1 CH₂Cl₂/EtOAc) yielded 2.84 g (43%) of compound 50 as a viscous oil: TLC R_{f} .35 (9:1 CH₂Cl₂/EtOAc); ¹H NMR (CDCl₃) 1.41 (m, 8H), 1.65 (m, 8H), 2.70 (two overlapping triplets, 4H), 3.08 (t, 2H), 3.65 (t, 2H), 3.81 (s, 6H), 6.85 (d, 4H), 7.32 (m, 7H), 7.47 (d, 2H); HRMS (FAB, M+), calc'd for C₃₃H₄O₄S₂: 568.2681. Found: 568.2665.

Compound 51- O-[14-(4',4"-Dimethyoxytriphenylmethoxy)-7,8-dithiotetradecyl]-O-(2-cyanoethyl)-N,N-diisopropylphosphoramidite: 458 mg (1.52 mmol) of O-cyanoethyl-N,N,N',N'-tetra-isopropylphosphorodiamidite in 0.5 mL of CH₂Cl₂ was added to a solution of 771 mg (1.36 mmol) of compound 50 and 116 mg (0.68 mmol) of diisopropylammonium tetrazolide in 6.8 mL of CH₂Cl₂ under N₂ atmosphere. The mixture was stirred for 4 hours and partitioned between 25 mL of NaHCO₃ and 3 x 25 mL of CH₂Cl₂. The combined CH₂Cl₂ layers were washed with saturated NaCl solution, dried (Na₂CO₃), filtered and concentrated to an oil. Purification by filtration through a 2" plug of basic alumina in a 25 mm column, eluting with 9:1 CH₂Cl₂/Et₃N provided 831 mg (80%) of compound 51 as a viscous oil: ¹H NMR (CDCl₃) d 1.25 (m, 12H), 1.45 (m, 8H), 1.70 (m, 8H), 2.72 (m, 6H), 3.09 (t, 2H), 3.65 (m, 4H), 3.87 (s, 6H), 3.91 (m, 2H), 6.89 (d, 4H), 7.35 (m, 7H), 7.49 (d, 2H); ³¹P NMR (CDCl₃ with 15% H₃PO₄ internal standard) 147.69; HRMS (FAB, MH+) calc'd for C₄₂H₆₂N₂O₅PS₂ 769.3839, found 769.3853.

Compound 52 - Trityl-HAD alcohol: 60 g (0.21 mol) of trityl chloride was added to a solution of 57 g (0.21 mole) of compound 49 and 60 mL of pyridine. This mixture was stirred at 100°C for 19 hours. The reaction mixture was cooled to room temperature and filtered. The filtrate was diluted with 300 mL of methylene chloride and extracted by 200 mL of saturated sodium bicarbonate. The organic layer was dried over Na₂SO₄, filtered and concentrated to an oil. Purification by silica gel chromatography (gradient 9:1 hexanes: ethyl acetate 3:1 hexanes:ethyl acetate) yielded 55 g of compound 52 (50%); ¹H NMR (CDCl₃) δ 1.38 (m, 8H), 1.63 (m, 8H), 2.66 (m, 4H), 3.04 (t, 2H), 3.62 (t, 2H), 7.25 (m, 9H), 7.42 (m, 6H). HRMS (FAB, M+) calc'd for C₃₁H₄₀O₂S 508.2470, found 508.2482.

Compound 53 - Trityl HAD Phosphoramidite: To a solution of 10 g (19.7 mmol) of compound 52 and 6.3 mL (36.2 mmol) of diisopropylethylamine in 90 mL of methylene chloride at 0°C under argon was slowly added 4.5 mL (20.2 mmol) of 2-cyanoethyl-N,N-diisopropylchlorophosphoramidite. After stirring for 90 minutes, the reaction mixture was extracted twice with 100 mL of saturated sodium bicarbonate. The methylene chloride solution was dried over Na₂SO₄, filtered and concentrated to an oil. Purification by basic alumina chromatography (75:24:1, hexanes:ethyl acetate:triethylamine) provided 11.3 g (81%) of compound 53 as an oil: ¹H NMR (CDCl₃) δ 1.18 (m, 12H), 1.37 (m, 8H), 1.62 (m, 8H), 2.6 (m, 6H), 3.04 (t, 2H), 3.60 (m, 4H), 3.82 (m, 2H), 7.26 (m, 6H), 7.44 (m, 9H). HRMS (FAB, MH+) calc'd for C₄₀H₅₈N₂O₃PS₂ 709.3626, found 709.3621.

Compound 54 - O-(tert-butyldimethylsilyl)-5-hexenol: 15.66 g (230 mmol) of imidazole and 20.0 g (130 mmol) of tert-butyldimethylsilyl chloride were added to a solution of 12.47 mL (10.4 g, 104 mmol) of 5-hexene-1-ol in 104 mL of DMF. The mixture was stirred at ambient temperature for 4 hours and partitioned between 200 mL of EtOAc and 100 mL of saturated NaHCO₃ solution. The EtOAc layer was washed with 100 mL of saturated NaHCO₃ solution, 100 mL of saturated NaCl solution, dried (MgSO₄), filtered, and concentrated to a volume of approximately 100 mL. Distillation under vacuum provided 70.07 g (90%) of compound 54: bp 130-143° @ 100 mm Hg; ¹H NMR (CDCl₃) 0.11 (s, 6H), 0.95 (s, 9H), 1.48 (m, 2H), 1.57 (m, 2H), 2.11 (dt, 2H), 3.66 (t, 2H), 5.03 (m, 2H), 5.86 (m, 1H); ¹³C NMR (CDCl₃) -5.25, 18.40, 25.21, 26.01, 32.35, 33.60, 63.09, 114.40, 138.92; Anal. calc'd for C₁₂H₂₆OSi: C, 67.22; H, 12.22. Found: C, 66.96; H, 12.16.

Compound 55 - 1-O-(tert-butyrldimethylsilyl)-1,5,6-hexanetriol: To a solution of 9.86 g (46.0 mmol) of compound 54 in 92 mL of acetone was added a solution of 6.46 g (55.2 mmol) of N-methylmorphoholine oxide in 23 mL of H₂O. To the mixture was added 443 µl of a 2.5% solution of OsO₄ in tert-butyl alcohol (360 mg of solution, 9.0 mg of OsO₄, 35 µmol) and 50 µL of 30% H₂O₂. The mixture was stirred for 16 h and a solution of 474 mg of sodium dithionite in 14 mL of H₂O was added. After another 0.5 h the mixture was filtered through celite. The filtrate was dried with MgSO₄ and filtered through 1" of silica gel in a 150 mL Buchner funnel using 250 mL portions of EtOAc to elute. Fractions containing product were concentrated to provide 11.0 g (96%) of 55 as a viscous oil: TLC R_{f} 0.2 (1:1 hexane/EtOAc); ¹H NMR (CDCl₃) 0.05 (s, 6H), 0.89 (s, 9H), 1.25 (m, 4H), 1.55 (m, 2H), 3.41 (dd, 2H), 3.62 (t, 2H), 3.71 (m, 1H); ¹³C NMR (CDCl₃) -5.23, 18.42, 21.91, 26.02, 32.68, 32.81, 63.16, 66.74, 72.24; HRMS (FAB, MH+), calc'd for C₁₂H₂₉O₃Si : 249.1886. Found : 249.1889.

Compound 56 - 5,6-(bis-O-benzoyl)-1-O-(tert-butyldimethylsilyl)-1,5,6-hexanetriol: 6.18 mL (7.48 g, 53.2 mmol) of benzoyl chloride was added to a solution of 5.29 g (21.3 mmol) of 55 in 106 mL of pyridine. The mixture was stirred for 18 hours and concentrated on the rotary evaporator. The mixture was partitioned between 100 mL of cold 1 N HCl and 100 mL of EtOAc. The pH of the aqueous layer was checked to make sure it was acidic. The EtOAc layer was washed successively with 100 mL of H₂O and 100 mL of saturated NaCl, dried (MgSO₄), filtered, and concentrated to provide 10.33 g (99%) of compound 56 as a viscous yellow oil: TLC R_{f} 0.45 (1:4 EtOAc/hexanes); ¹H NMR (CDCl₃) d 0.05 (s, 6H), 0.88 (s, 9H), 1.59 (m, 4H), 1.85 (m, 2H), 3.14 (t, 2H), 4.49 (dd, 1H), 4.59 (dd, 1H), 5.54 (m, 1H), 7.45 (m, 4H), 7.58 (m, 2H), 8.05 (m, 4H).

Compound 57 - 5,6-(bis-O-benzoyl)-1,5,6-hexanetriol: 10.7 mL (10.7 mmol) of 1 N tetrabutylammonium fluoride in THF was added to a solution of 2.62 g (5.36 mmol) of compound 56 in 10.9 mL of THF. The mixture was stirred for 16 hours. The mixture was partitioned between 25 mL of saturated NaHCO₃ solution and 3 x 25 mL of EtOAc. The combined EtOAc extracts were washed with saturated NaCl solution, dried (MgSO₄), filtered and concentrated to a viscous oil which was purified by silica gel chromatography (1:1 hexane/EtOAc) to provide 823 mg (41%) of compound 57 as a viscous oil; R_{f} 0.14 (1:1 hexane/EtOAc); ¹H NMR (CDCl₃) d 1.58 (m, 2H), 1.68 (m, 2H), 1.88 (m, 2H), 3.68 (t, 2H), 4.52 (dd, 1H), 4.62 (dd, 1H), 5.56 (m, 1H), 7.46 (m, 4H), 7.58 (m, 2H), 8.05 (m, 4H); ¹³C NMR (CDCl₃) d 22.08, 31.20, 31.30, 32.88, 62.92, 66.17, 72.63, 128.93, 130.19, 130.57, 133.62, 166.72, 166.86; HRMS (FAB MH+), calc'd for C₂₀H₂₃O₅; 343.1545. Found: 343.1553.

Compound 58 - O-[5,6-(bis-O-benzoyloxy)-hexyl]-O-(2-cyanoethyl)-N,N-diisopropylphosphoramidite: A solution of 989 mg (3.28 mmol) of O-cyanoethyl-N,N,N', N'-tetraisopropylphosphorodiamidite in 2.0 mL of CH₂Cl₂ was added to a solution of 1.02 g (2.98 mmol) of compound 57 and 255 mg (1.49 mmol) of diisopropylammonium tetrazolide (prepared by mixing acetonitrile solutions of diisopropylamine and tetrazole in a one-to-one mole ratio and concentrating to a white solid) in 14.9 mL of CH₂Cl₂. The mixture was stirred for 4 hours and then partitioned between 25 mL of CH₂Cl₂ and 25 mL of chilled saturated NaHCO₃ solution. The CH₂Cl₂ layer was washed with saturated NaCl solution, dried (Na₂SO₄), filtered, and concentrated. Purification by filtration through a 2" plug of basic alumina in a 25 mm column, eluting with 9:1 EtOAc/Et₃N, provided 1.5 g (93%) of compound 58 as a viscous oil: ¹H NMR (CDCl₃) d 1.19 (m, 12H), 1.62 (m, 2H), 1.73 (m, 2H), 1.90 (m, 2H), 2.62 (dd, 2H), 3.53-3.92 (m, 6H), 4.53 (dd, 1H), 4.62 (dd, 1H), 5.58 (m, 1H), 7.48 (m, 4H), 7.60 (m, 2H), 8.09 (m, 4H); ³¹P NMR (CDCl₃ with 15% H₃PO₄ internal standard) d 148.2; HRMS (FAB, MH+), calc'd for C₂₉H₄₀O₆N₂P 543.2624. Found, 543.2619.

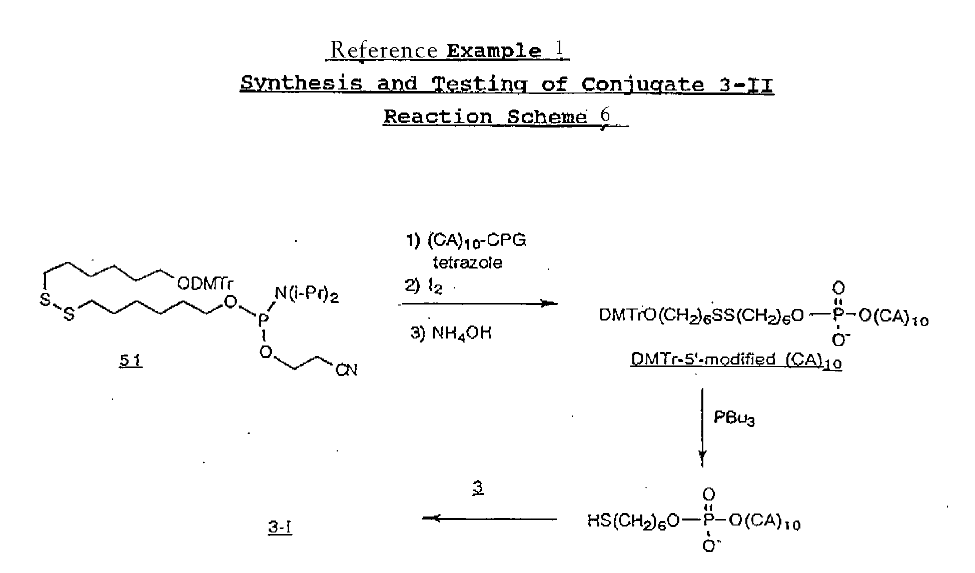

### Preparation of DMTr-5'-Modified (CA)₁₀.

The polynucleotide d-[DMTr-(bzCp(CE)bzA)₁₀] was prepared on a Milligen 8800 Prep Scale DNA synthesizer following the manufacturer's protocols for DNA phosphoramidite synthesis. The synthesis was carried out on 10 g of DMTr-d-bzA-CPG support with a nucleoside loading of 30.0 µmol/g. The final DMTr blocking group was removed using the machine protocol. Milligen activator solution, Cat. No. MBS 5040 (45 mL) and 0.385 g of compound 51 (see Reaction Scheme 4) were added to the reaction and the suspension was mixed for 8 minutes by argon ebullition. The mixture was oxidized by the usual machine protocol and the support-bound polynucleotide was collected by filtration, air dried and treated with 100 mL of concentrated ammonia for 16 hours at 55°C. When cool, the mixture was filtered through a Gelman 10*µ* polypropylene filter. The filter was washed with 200 mL of 2 mM NaCl adjusted to pH 12 with NaOH. The filtrate was then applied to a Amicon chromatography column (0.45 x 9.4 cm, 150 mL) which had been packed with Q-Sepharose (Pharmacia) equilibrated first with 3M NaCl and then with 2 mM NaCl, pH 12. The column was eluted with 500 mL of a linear gradient (2 mM NaCl, pH 12 to mL 1.3 M NaCl, pH 12), then washed with 1.3 M NaCl, pH 12 until all U.V. absorbing material came off. Fractions which absorbed at 260 nm were further analyzed by polyacrylamide electrophoresis and those containing pure product were pooled. The pool (120 mL) was treated with 240 mL of cold isopropanol and stored for 30 minutes at - 20°C. The precipitate was collected by centrifugation in a Sorvall RC 3B centrifuge using a model H-6000A rotor for 15 minutes at 3000 rpm and 4° C to yield DMTr-5'-modified (CA)₁₀ (14946 A₂₆₀ units, 498 mg, 62.2 µM, 20% based on 300 µM CPG nucleoside.)

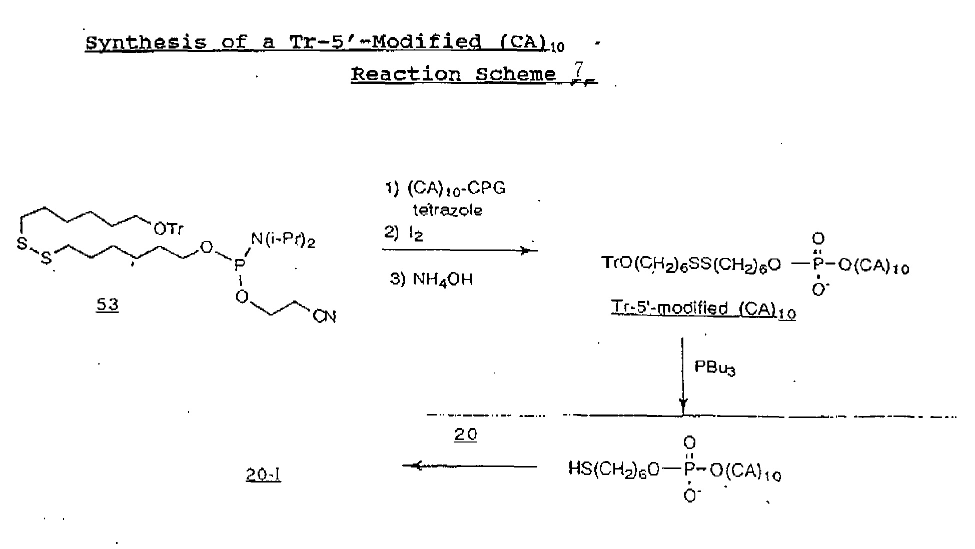

The synthesis of Tr-5'-modified (CA)₁₀ was carried out as described above for the synthesis of DMTr-5'-modified (CA)₁₀ (prepared as described in Reaction Scheme 4) by substituting compound 53 for compound 51.

### conjugation of DMTr-5'Modified Polynucleotides to Compound 3 (TA-DABA-PEG, Reaction Scheme 1) - Preparation of Conjugate 3-I

In the conjugation procedures that follow, all the buffers and solutions employed were thoroughly sparged with helium and all reaction vessels were purged with argon before use. A solution of 11,568 A₂₆₀ units (48.2 µmol, assume molar extinction at 260 nm = 240,000) of the DMTr-5'-modified (CA)₁₀ in 7.7 mL water was treated with 1 mL of 0.1 M NaHCO₃ and 210 µL (876 µmol, 18 times molar excess) tributylphosphine for 0.5 hour at room temperature. The suspension was shaken from time to time. The suspension was treated with 0.8 mL of 3M NaCl and 16 mL of cold isopropanol. After 30 minutes at -20°C, the material was centrifuged at 3000 rpm for 20 minutes. The pellet was redissolved in 2 mL of water, 0.2 mL of 3M NaCl, treated with 4 mL isopropanol and recentrifuged. The pellet was briefly dried under vacuum and dissolved in 2.8 mL of water and 1 mL of 0.1 N NaHCO₃ which had been sparged with helium. 6.7 mg of compound 3 (IA-DABA-PEG) was added, and the mixture was kept for 16 hours at room temperature in the dark. The reaction mixture in a final volume of 6 mL was applied to a 5 x 91 (1800 Ml) Pharmacia column which was packed with Sephacryl 200 (Pharmacia). The column was eluted with 0.5 M NaCl, 0.1 M sodium borate, pH 8.3. A peristaltic pump was used and set to give a flow rate of approximately 2 mL per min., and fractions of 15 ml were collected. The absorbance of the fractions at 260 nm was measured. The fractions were also analyzed by polyacrylamide gel electrophoresis and those containing pure conjugate were pooled.

### Hybridization of Conjugate 3-I- Preparation of Conjugate 3-II

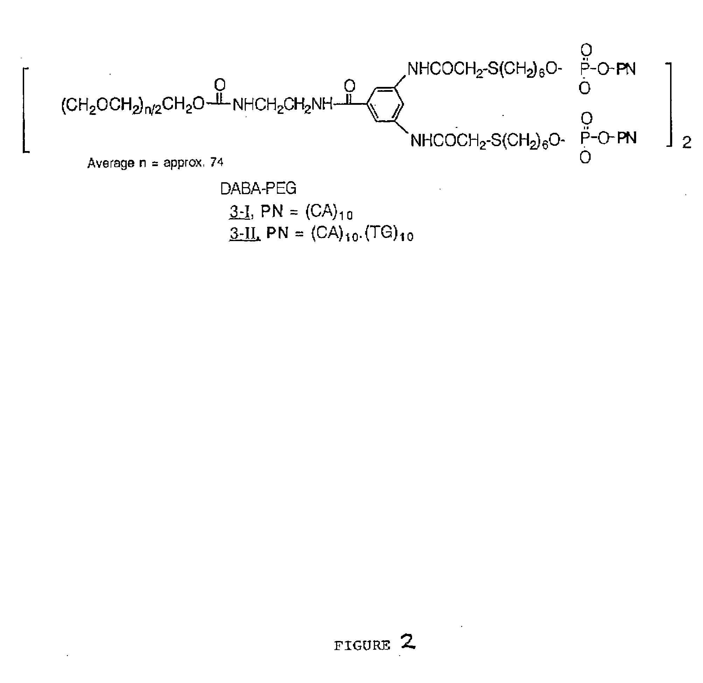

The pooled fractions from above contained 726 A₂₆₀ units. The equivalent amount of (TG)₁₀ was added and the tube was heated at 90°C for ten minutes and then allowed to cool to room temperature over 1.5 hours. An equal amount of isopropanol was added and the mixture kept for 3 hours at -20°C. After centrifugation at 3000 rpm for 20 minutes, the pellet was dissolved in 0.15 M NaCl, 0.01 M sodium citrate, pH 6.8. 53 mg of the hybrid was obtained. An aliquot of the material was diluted in the above buffer and the melting temperature of the duplex was determined in a carey 3E spectrophotometer. The material had a Tm of 73.4°C. and 24.3% hyperchromicity. A 10 A₂₆₀ unit aliquot of the product was annealed with excess (TG)₁₀ as described above. This as well as unannealed conjugate and a (TG)₁₀ standard were analyzed by gel permeation HPLC on a Shodex Protein KW 8025 column on a Rainin HPLC instrument. The column was eluted isocratically with 0.05M NaH₂PO₄, pH 6.5, 0.5M NaCl. The run time was 12 minutes. The product had a retention time of 6.9 minutes and (TG)₁₀ 9.2 minutes. Comparison of the area under the peaks showed that 98.09% of the product was double stranded DNA. The conjugate is represented by the structure designated "Conjugate 3-II" in Figure 2.

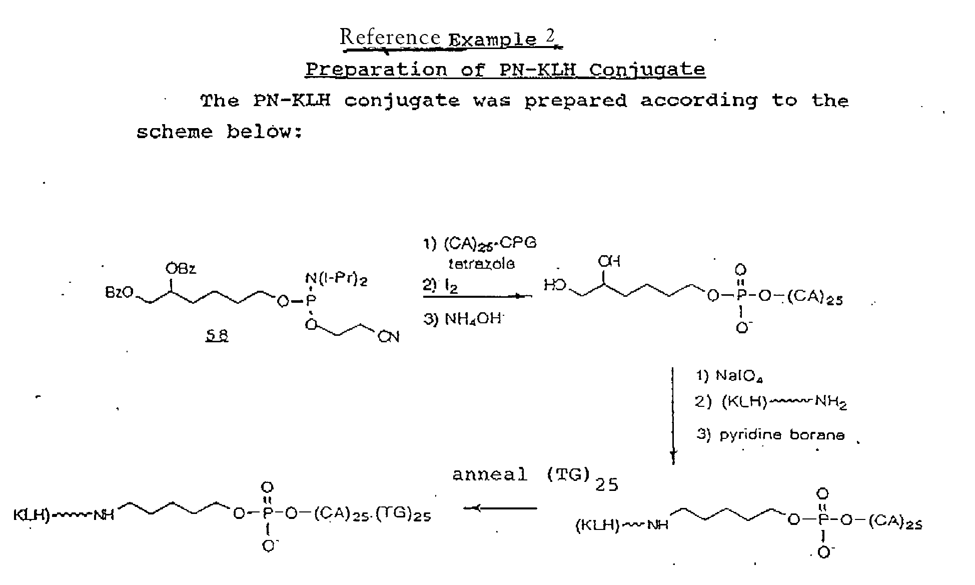

### synthesis of ACT-Modified (CA)₂₅

Compound 58 was coupled to (CA)₂₅ as the final step of automated synthesis. Forty-nine sequential steps were carried out using alternating dC and dA phosphoramidites beginning with 10 g of DMT-d-bzA-CPG support with a nucleoside loading of 30 µmol/g. The DMTr blocking group was removed from the resulting d-[DMTr-(bzCp(CE)bzA)₂₅], and 40 mL of activator solution (Milligen, Cat. No. MBS 5040) and 800 mg of compound 58 were added to the reaction mixture. The suspension was mixed for 8 minutes by argon ebullition and subjected to a conventional oxidation step. The support bound polynucleotide was removed from the reaction vessel, air dried, and treated with 100 mL of concentrated ammonia for 40 hours at 55°. When cool, the mixture was filtered through a Gelman 10 µm polypropylene filter and the filtrate was then purified by conventional ion exchange chromatography. Fractions which absorbed at 260 nm were further analyzed by polyacrylamide gel electrophoresis and those containing pure product were combined and precipitated with isopropanol to provide 510 mg (31.9 *µ*mol, 10%) of the ACT-modified (CA)₂₅.

### synthesis of Single-stranded PN-KLH Conjugate

To a solution of 100 mg (2.5 *µ*mol) of NaIO₄-treated, ACT-modified (CA)₂₅ in 1.33 mL of 50 mM sodium borate pH 8.0 was added 31.3 mg (0.208 *µ*mol) of KLH and 2.0 mg (31.8 *µ*mol) of pyridine-borane. The mixture was kept at 37°C for 72 h, and the product was purified by chromatography on S-200.

### Hybridization of Single-stranded PN-KLH Conjugate with (TG)₂₅

The equivalent amount of (TG)₂₅ was added to the single-stranded PN-KLH conjugate and the tube was heated at 90°C for ten minutes and then allowed to cool to room temperature over an hour and a half. Precipitation with isopropyl alcohol yielded 53 mg of product, PN-KLH; Tm (0.15 M NaCl, 0.01 M sodium citrate, pH 6.8) 73.4°, 31.1% hyperchromicity; 98% double stranded as determined by HPLC comparison to standards consisting of sample annealed with excess (TG)₁₀, unannealed conjugate, and unannealed (TG)₁₀ (Shodex Protein KW 8025 column, 0.05 M NaH₂PO₄, pH 6.5, 0.5 M NaCl). This conjugate may be represented by the formula

KLH-[NH(CH₂)₅OPO₂, O-(CA)₂₅: (TG)₂₅]₋₅

(assuming a molecular weight of 10⁵ for KLH) and is designated "PN-KLH."

### Testing of Conjugate 3-II as a Tolerogen

Conjugate 3-II was tested for its ability to tolerize mice that had been immunized with an immunogenic form of the polynucleotide.

### Material and Methods

Mice: C57BL/6 female mice 6 weeks of age were purchased from Jackson Laboratories, Bar Harbor, ME. The mice were housed and cared for by NIH approved methods.

Immunization: The mice were primed, according to the method of Iverson (Assay for *in vivo* Adoptive Immune Response in Handbook of Experimental Immunology, Vol. 2 Cellular Immunology, Eds. D.M. Weir, L.A. Herzenberg, C. Blackwell and A. Herzenberg, 4th Edition, Blackwell Scientific Publications, Oxford) by injecting the mice, i.p., with 100 *µ*g of PN-KLH precipitated on alum and with 2 x 10⁹ formalin fixed pertussis organisms as an adjuvant. The mice were boosted with 50 *µ*g of PN-KLH, in saline, i.p.

Coupling of PN to SRBC: Sheep Red Blood Cells (SRBC) in Alsevers were purchased from Colorado Serum Co., Denver, CO, and used within two weeks. The SRBC were coated with (CA)₂₅:(TG)₂₅ (a 50 mer of CA:GT) by the method of Kipp and Miller ("Preparation of Protein-Conjugated Red Blood Cells with ECDI (Modification)" in Selected Methods in Cellular Immunology, (1980), Eds. B.B. Mishell and S.M. Shiigi, W.H. Freemen and Co., San Francisco, p. 103). Briefly, the SRBC were washed 4 times in cold saline, mixed with 2 mg of (CA)₂₅:(TG)₂₅ coupled to D-EK in 0.35M mannitol, 0.01 M NaCl containing 10 mg of carbodiimide and incubated for 30 minutes at 4°C. The coated SRBC were washed twice with cold Balanced Salt Solution and resuspended to 10% (v/v).

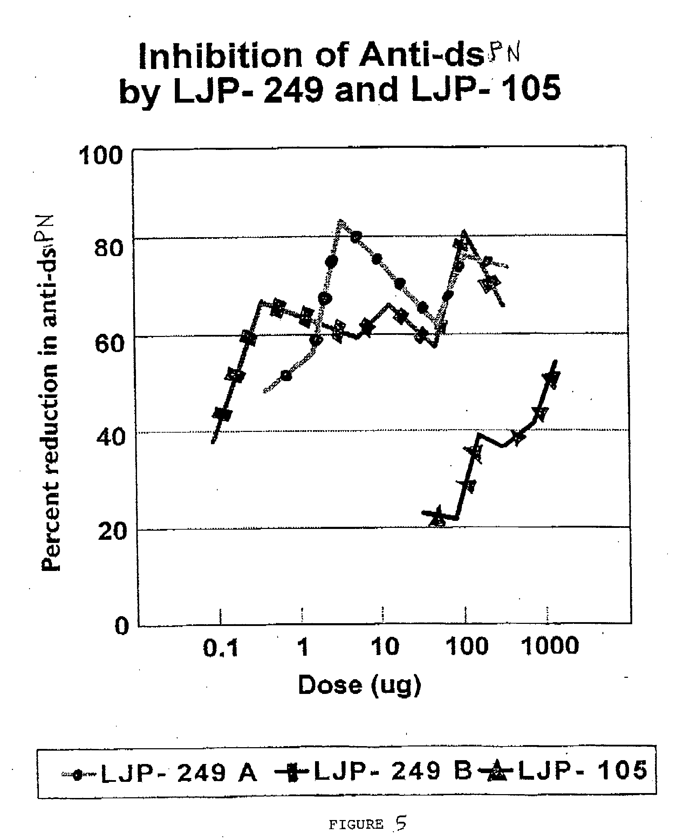

Plaque assay: The number of anti-PN plaque forming cells (pfc) was determined using the Cunningham technique (Marbrook, J., "Liquid Matrix (Slide Method)", in Selected Methods in Cellular Immunology, (1980), Eds. B.B. Mishell and S.M. Shiigi, W.H. Freemen and Co., San Francisco, p. 86.) The number of IgG pfc were determined by elimination of IgM plaques using rabbit and anti-mouse IgG as described by Henry ("Estimation of IgG responses by Elimination of IgM Plaques" in Selected Methods in Cellular Immunology, (1980), Eds. B.B. Mishell and S.M. Shiigi, W.H. Freemen and Co., San Francisco, p. 91). Briefly, spleens were harvested and single cell suspensions made in balanced salt solution (BSS). Guinea pig serum was added to polynucleotide coated SRBC to give a final dilution of 1:9 guinea pig serum, and enough rabbit anti-mouse IgG was added to give a final dilution of 1:100 rabbit anti-mouse IgG. Equal volumes of the SRBC mixture and diluted spleen cells were mixed in microtiter wells and transferred to Cunningham chambers. Each spleen was tested individually and in triplicate. The edges of the chambers were sealed with paraffin and the chambers were incubated at 37°C for 1 hour. The number of plaques were enumerated by viewing the chambers under an inverted microscope. The percentage reduction of anti-dsDNA antibodies is illustrated in Figure 5.

### Results

Mice were primed with PN-KLH precipitated on alum with pertussis as an adjuvant (A&P) and seven weeks later divided into groups of 3 mice each. The mice were treated, i.p., with doubling dilutions of PN-DABA-PEG, Conjugate 3-II five days later all of the mice, including the control, were boosted with 50 µg of PN-KLH, in saline, i.p. Four days later, the spleens were harvested and the number of IgG pfc determined. As shown in Table 1, all doses of Conjugate 3-II tested showed a significant reduction in the number of pfc as compared to the control group.

**Table 1**

| Tolerogenic Activity of Conjugate 3-II (PN-DABA-PEG) | | |
|---|---|---|
| Dose (µg/mouse) | pfc/10⁶ spleen cells Mean (S.D.) | % Reduction Mean |
| None | 12865 (2846) | |
| 62.5 | 2868 (6809) | 77.7 |
| 125 | 3331 (939) | 74.1 |
| 250 | 3044 (1929) | 76.3 |
| 500 | 1809 (759) | 85.9 |
| 1000 | 2814 (554) | 78.1 |

### Example 3

### Preparation and Testing of Conjugate 20-II

### Conjugation of 5' Modified (CA)₁₀ to Valency Platform Molecule 20 - Preparation of Single-Stranded Conjugate 20-I

969 µL (789 mg, 3.89 mmol) of tri-n-butylphosphine was added to a solution of 918 mg (0.14 mmol) of 5'-modified (CA)₁₀ in 30 mL of H₂O under argon atmosphere. The mixture was stirred for 1 hour and then 2.4 mL of a 3M NaCl solution was added followed by 42 mL of isopropanol which had been sparged with helium to remove oxygen. The mixture was placed in a freezer at - 20°C for 1 hour and then centrifuged at 3000 rpm for 30 minutes. The supernatant was removed and the oily residue was dissolved in 15.5 mL of helium sparged H₂O. 1.24 mL of 3M NaCl and 21.7 mL of helium sparged isopropanol was added to the mixture. The resulting mixture was then placed in a freezer at -20°C for 1 hour and centrifuged at 3000 rpm for 20 minutes. The oily pellet was dried under vacuum for 18 hours to yield a solid. The solid was dissolved in 6 mL of helium sparged H₂O to give a total volume of 6.4 mL. The amount of DNA was 863 mg as determined by UV absorbance at 260 nm (0.033 mg per unit of absorbance in pH 7.5 phosphate buffered saline). The solution was transferred to a 50 mL three-neck flask under argon. One neck of the flask had an argon gas inlet while the other two necks were stoppered. The total volume was adjusted to 7.7 mL with H₂O and 0.87 mL of helium sparged 1M sodium phosphate buffer, pH 7.8, and 0.97 mL of MeOH. 1.9 mL (33.63 mg, 0.025 mmol) of a 17.7 mg/mL solution of compound 20 in MeOH was added to the mixture. The resulting mixture was stirred under argon for 20 hours and then diluted to 100 mL with a solution comprising 0.1 M NaCl, 0.05 M sodium phosphate, pH 7.5, and 10% MeOH. Purification was accomplished by chromatography on Fraatogel® (equilibration: 0.1 M NaCl, 0.05 M sodium phosphate, pH 7.5, 10% MeOH: elution gradient 0.5 M NaCl, 0.05 M sodium phosphate, pH 7.5, 10% MeOH to 0.8 NaCl, 0.05 M sodium phosphate, pH 7.5, 10% MeOH). Fractions containing pure conjugate 20-I as evidenced by HPLC and polyacrylamide gel electrophoresis were collected in 232 mL of eluent. The product and salts were precipitated by adding an equal volume of isopropanol and placing same in a freezer at -20°C for 1 hour. Dialysis against H₂O (2 x 100 vol) gave 335 mg of conjugate 20-I (32 mL of 10.47 mg/mL, 0.033 mg/absorbance unit at 260 nm).

### Annealing of Conjugate 20-I with (TG)₁₀ to Form Double-Stranded Conjugate 20-II

150 mg (14.33 mL of 10.47 mg/mL based on 0.033 mg/absorbance unit at 260 nm) of conjugate 20-I and 157.5 mg (1.50 mL of 104.6 mg/mL based on 0.033 mg/absorbance unit at 260 nm) of (TG)₁₀ were placed into a 50 mL polypropylene centrifuge tube. The concentration was adjusted to 15 mg/mL by adding 2.0 mL of pH 7.2 10X PBS and 2.17 mL of H₂O. The mixture was placed in a 90°C water bath and allowed to cool to room temperature over 1.5 hours. The concentration was determined to be 17.7 mg/mL by absorbance at 260 nm (0.050 mg/absorbance unit); transition melt temperature 67.5°C; hyperchromicity 27%; osmolality 346; pH 7.2. For final formulation of conjugate 20-II, the solution was diluted to a final concentration of 12.7 mg/mL and an osmolality of 299 by adding 7.23 mL of pH 7.2 ½ X PBS and filtering through a 0.22 µ filter.

### Alternative Conjugation of 5'-Modified (CA)₁₀₋₂₀, Conjugate 20-I

10 equivalents of tri-n-butylphosphine are added to a 10 mg/mL solution of 5'-modified (CA)₁₀ in He sparged with 100 mM pH 5 sodium acetate. The mixture is stirred for 1 hour and then precipitated with 1.4 volumes of isopropyl alcohol (IPA). The mixture is placed in the freezer at -20°C for 1 hour and centrifuged at 3000 rpm for 20 minutes. The supernatant is removed and the pellet is dissolved to 10 mg/mL in He sparged IPA. The mixture is placed in the freezer at -20°C for 1 hour and centrifuged at 3000 rpm for 20 minutes. The pellet is dried under vacuum for 18 hours to give a solid. A 50 mg/mL solution of the solid is prepared in He sparged 100 mM pH 10 sodium borate buffer. 0.25 equivalents of compound 20 as a 40 mg/mL solution in 9/1 MeOH/H₂O is added to the mixture. The mixture is stirred at room temperature for 3-20 hours and diluted (0.1 M NaCl, 0.05 sodium phosphate, pH 7.5, 10% MeOH). Purification is accomplished by chromatography on Factogel (equilibration; 0.1 M NaCl, 0.05 M sodium phosphate, pH 7.5, 10% MeOH: elution gradient; 0.5 M NaCl, 0.05 M sodium phosphate, pH 7.5, 10% MeOH to 0.8 M NaCl, 0.05 sodium phosphate, pH 7.5, 10% MeOH). Fractions containing pure 20-I, as evidenced by HPLC and polyacrylamide gel electrophoresis, were collected. The product and salts are precipitated by adding an equal volume of IPA and standing in the freezer at -20°C for 1 hour. Dialysis against H₂O (2 X 10 vol) give 20-I.

### Alternative Annealing of 20-I with (TG)₁₀₋₂₀ to Form Double Stranded Conjugate 20-II

The methodology is essentially the same as that described above except that annealing is done at 70°C instead of 90°C.

### Second Alternative Conjugation of 5'-Modified (CA)₁₀₋₂₀. Preparation of Single Stranded Conjugate 20-I

4.8 mL of tri-n-butylphosphine was added to a solution of 7.75 g of 5'-modified (CA)₁₀ in 104 mL of Ar sparged 100 mM pH 5 sodium acetate under N₂. The mixture was stirred for 1 hour and then precipitated with 232.5 mL of IPA. The mixture was placed in a freezer for -20°C for 1.5 hours, centrifuged at 3000 rpm for 20 minutes and then frozen at -20°C for 24 hours. The supernatant was removed and the pellet was dissolved in 170 mL He sparged 0.3 M NaCl solution. The mixture was again precipitated with 232 mL of Ar sparged IPA. The mixture was then placed in a freezer at -20°C for 2 hours, centrifuged at 3000 rpm for 20 minutes and then from at -20°C for 11 hours. The supernatant was decanted and the pellet was dried under vacuum for 12 hours to give a solid. A solution of the solid was prepared in 110 mL of Ar sparged 100 mM pH 10 sodium borate buffer. 406 mg of compound 20 as a solution in 4.4 mL of 9/1 MeOH/H₂O was added to the mixture. The mixture was stirred at room temperature for 2 hours. The product mixture contained 62% of 20-I by high-pressure ion chromatography, Waters Gen Pak Fax column (100 X 4 mm), 60°C, linear gradient from 65%A/35%B to 18%A/82%B; A=0.05 M NaH₂PO₄, pH 7.5, 1 mM EDTA, 10% MeOH (v/v); B=0.05 M NaH₂PO₄, pH 7.5, 1 M NaCl, 1 mM EDTA, 10% MeOH (v/v), eluting at 19.5 minutes.

### Testing of Conjugate 20-II and Nonconjugated Controls

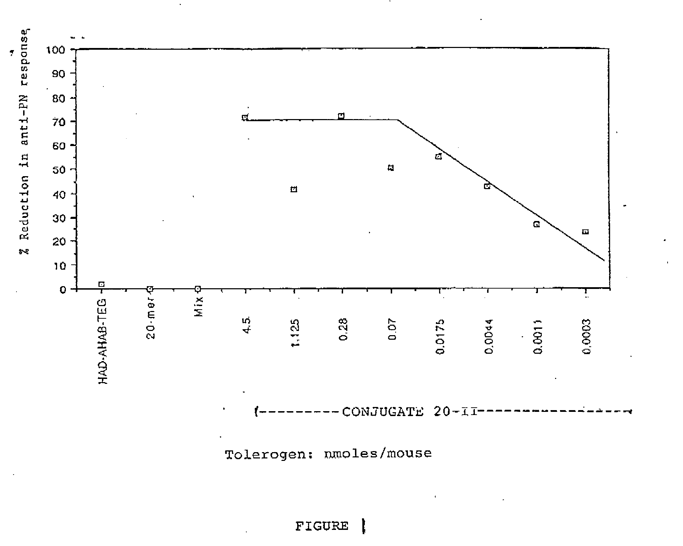

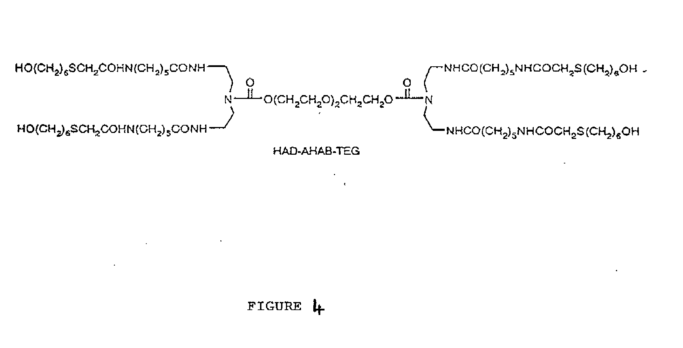

C57BL/6 mice were immunized with PN-KLH and A&P. After three weeks, groups of 5 mice/group were treated with either different doses of Conjugate 20-II or 4.5 nM HAD-AHAB-TEG (linker, HAD, attached to derivatized valency platform molecule, AHAB-TEG, see Figure 4), or 1,8 nM (4 X 4.5) (CA)₁₀: (TG)₁₀, or a mixture of 4.5 nM HAD-AHAB-TEG plus 18 nM (CA)₁₀: (TG)₁₀, i.p.; and one group was not treated. The groups were given booster injections and the sera were collected and assayed as described in Reference Example 2. The percent reduction of the anti-PN response is shown in Figure 1. The anti-KLH responses of these mice was normal. The results clearly show that the anti-PN response was not affected by (i) the valency platform molecule alone, (ii) the PN alone, or (iii) a mixture of the two. The PN must be coupled to the nonimmunogenic valency platform molecule in order to induce tolerance.

### Conjugate 20-II Causes a Reduction in the Number of PN-specific Antibody Producing Cells

C57B1/6 mice were immunized with PN-KLH, A&P. After three weeks, groups of 3 mice/group were treated with different doses of Conjugate 20-II, i.p; one group was not treated. After five days, all of the mice were given a booster injection of PN-KLH in saline, i.p., and then 4 days later their spleens were harvested and assayed for the number of PN-specific, IgG-producing cells using the hemolytic plaque assay. The results, shown in Table 2, clearly show that this conjugate reduced the number of PN-specific IgG-producing cells.

**TABLE 2**

| REDUCTION IN THE NUMBER OF pfc BY Conjugate 20-II | | | |
|---|---|---|---|
| Group# | Dose µg/mouse | PN-specific pfc per 10⁶ spleen cells (Mean & S.E.) | % Reduction |
| 1 | None | 5562 (2570) | |
| 2 | 274 | 982 (1871) | 82.3 |
| 3 | 91 | 1867 (1335) | 66.4 |
| 4 | 30 | 2247 (1606) | 59.6 |
| 5 | 10 | 6109 (2545) | 0 |
| 6 | 3 | 4045 (1411) | 27.3 |
| 7 | 1 | 4578 (2475) | 17.7 |
| 8 | 0.4 | 5930 (897) | 0 |

### EXAMPLE 4

### TREATMENT OF BXSB MICE WITH LJP 394, CONJUGATE 20-II

### Mice Treatment Protocol

Six to 9 week old male BXSB mice (Jackson Laboratory, Bar Harbor, Me) were housed at the La Jolla Pharmaceutical facility. Food and water were provided as libitum. Animals were rested one week prior to use. Initial blood samples and weights were obtained prior to the first conjugate treatment. Conjugate treatment was initiated at 7 to 9 weeks of age and was administered intravenously twice weekly from day 59 to day 150. Animals were bled periodically and their anti-DNA antibody titers were determined.

### Assay for IgG Anti-DNA Antibody Production

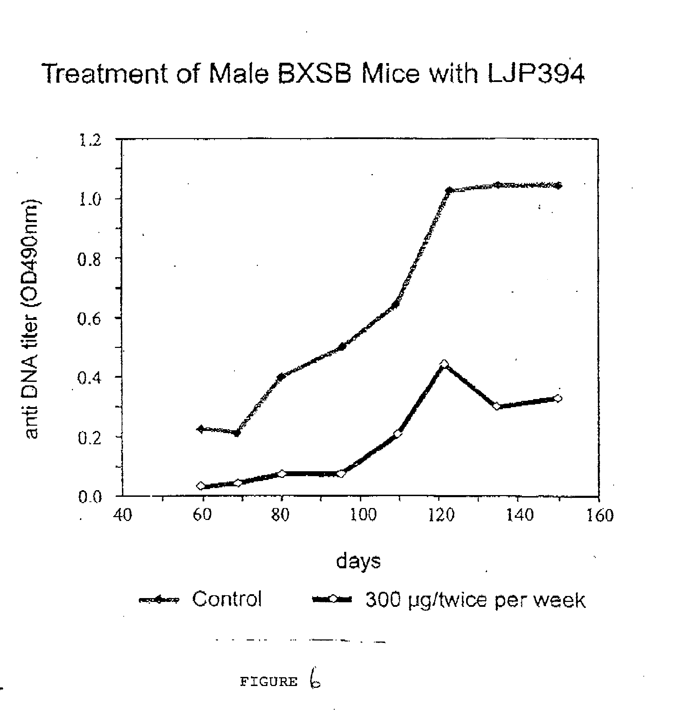

A serum sample taken from each mouse was assessed for the presence of anti-DNA antibody by ELISA. Falcon Probind 96 well microtitration assay plates (Becton Dickerson, Oxnard, CA) were coated with 100 µL/well of (PN)₅₀-D-EK (a co-polymer of D-glutamic acid and D-lysine) at a concentration of 50 µg/mL overnight at 4°C. The plates were washed twice with PBS without calcium and magnesium and 0.05% Tween 20 (wash buffer) using a M96V plate washer (ICN Biomedical, Inc., Irvine, CA). Plates were blocked for 1 hour at room temperature in PBS containing 1% gelatin (Norland Products, Inc., New Brunswick, NJ) and 0.05% Tween 20. Plates were washed twice with wash buffer before the addition of serum samples or standards. Serum samples and standards were prepared in a diluent containing PBS with 1% gelatin, 0.05% Tween 20 and 10% goat serum. Plates were incubated with serum samples for 60 to 90 minutes at 37°C and then the wells were washed four times with wash buffer. Biotinylated goat anti-mouse IgG (Sigma Chemical Co., St. Louis, MO) was diluted 1/1000 in blocking solution containing 10% goat serum. The plates were incubated for 1 hour at 37°C and washed four times. The substrate, OPD (Sigma Chemical Co., St. Louis, MO),was added. The plates were incubated in the dark until the highest reading of the highest standard was approximately 1 OD unit by an ELISA plate reader at OD 450 nm (Bio-Tek Instruments, Winooski, VT). The reaction was stopped with 50 µL of 3M HCl and the plates were read at 490 nm. The reference positive serum was included in each microtitration plate and the positive wells from each assay were within the sensitivity range of the reference curve 95% of the time. In the later bleeds, some positive samples exceeded the reference curve. However the most dilute mouse serum sample was within the range of the reference curve. No significant binding was observed by normal control negative serum. The results are shown in Figure 6.

## Claims

1. A conjugate comprising a polynucleotide coupled to a valency platform molecule, which conjugate has the general formula (I): 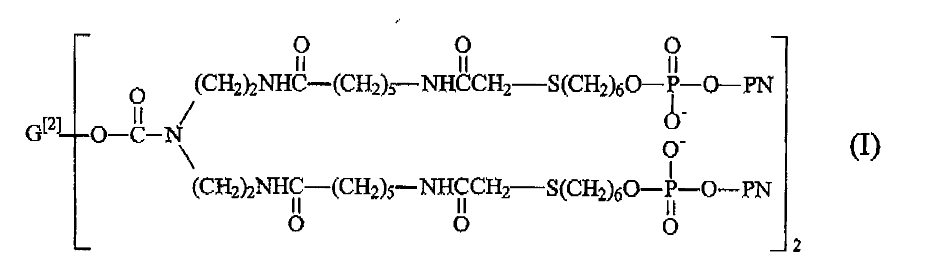 wherein G^{[2]} is -CH₂H₂OCH₂)ᵣCH₂- wherein r = 0 to 300 and PN is a polynucleotide.

2. A conjugate according to claim 1, wherein G^{[2]} is:
-CH₂CH₂OCH₂CH₂OCH₂CH₂-

3. A conjugate according to claim 1 or 2, wherein the polynucleotide is DNA.

4. A conjugate according to any one of the preceding claims, wherein the polynucleotide is a polynucleotide duplex of at least 20 base pairs.

5. A conjugate according to claim 4, wherein the duplex is 20 to 50 base pairs in length.

6. A conjugate according to claim 4, wherein the polynucleotide is (CA)₁₀:(TG)₁₀.

7. A conjugate according to any one of claims 4 to 6, wherein the polynucleotide duplexes are bound to the valency platform molecule at or proximate one of the ends of each duplex.

8. A conjugate according to any one of claims 4 to 7, wherein the polynucleotide duplex has a significant binding activity for human systemic lupus erythematosus anti-dsDNA autoantibodies.

9. A conjugate according to claim 1, which has the formula:

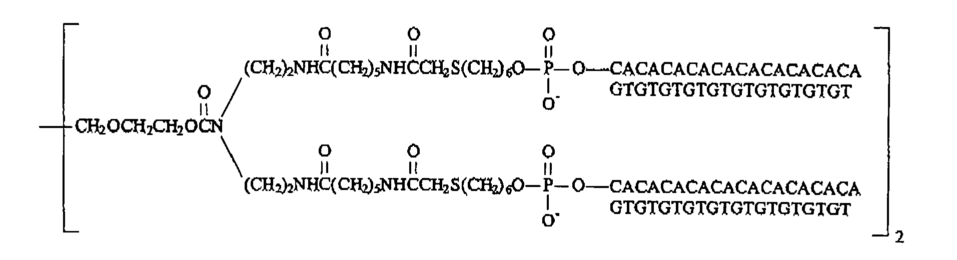

10. A conjugate according to claim 1, wherein the valency platform molecule is obtainable by reacting polyethylene glycol-bis-chloroformate with N,N-di(2-[6'-N'-carbobenzyloxyaminohexanoamido]ethyl)amine.

11. A method for making a conjugate as defined in claim 4, which method comprises:
(a) conjugating a polynucleotide-linker of general formula (II): 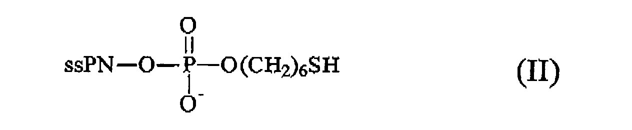 wherein ssPN denotes a single-stranded polynucleotide of at least 20 nucleotides in length, to a valency platform molecule of the general formula (III): 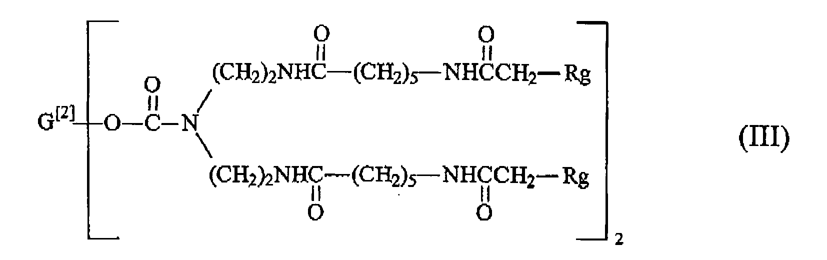 wherein Rg is halogen and G^{[2]} is as defined in claim 1; and
(b) annealing complementary single-stranded polynucleotides to each said single-stranded polynucleotide ssPN.

12. A method according to claim 11, wherein Rg is bromine.

13. A method according to claim 11 or 12, wherein the single-stranded polynucleotide is (CA)₁₀ and the complementary single-stranded polynucleotide annealed thereto is (TG)₁₀, such that the conjugate comprises the double-stranded polynucleotide:

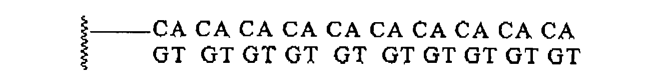

14. A pharmaceutical composition comprising a conjugate as defined in any one of claims 1 to 10 and a pharmaceutically acceptable vehicle.

15. A composition according to claim 14 wherein the vehicle is a pharmaceutically acceptable injectable aqueous carrier.

16. A conjugate as defined in any one claims 1 to 10 for use in a method of treatment of the human or animal body by therapy.

17. A conjugate as defined in claim 8 or 9 for use in a method of treating lupus.

18. A valency platform molecule of the general formula (III): 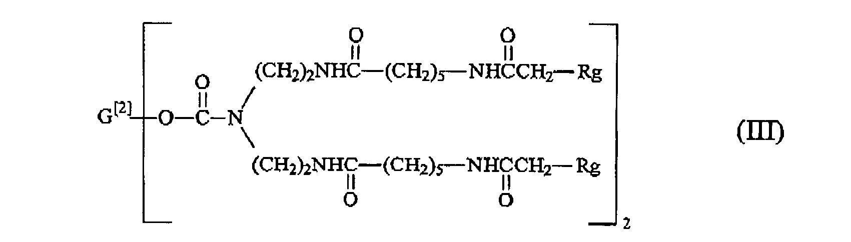 wherein G^{[2]} is -CH₂(CH₂OCH₂)ᵣCH₂- wherein r = 0 to 300 and Rg is halogen.

19. A valency platform molecule according to claim 18, wherein G^{[2]} is:
-CH₂CH₂OCH₂CH₂OCH₂CH₂-.

20. A valency platform molecule according to claim 18 or 19, wherein Rg is bromine.

21. A valency platform molecule according to claim 18, which has the formula:

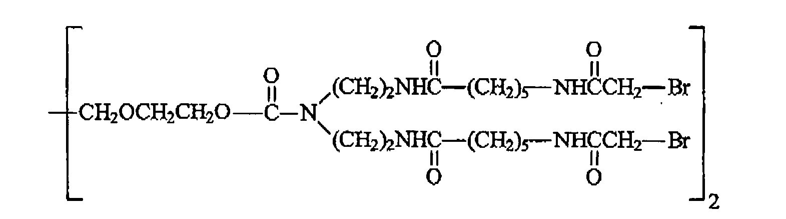

22. A valency platform molecule according to claim 18, which is obtainable by reacting polyethylene glycol-bis-chloroformate with N,N-di(2-[6'-N'-carbobenzyloxyaminohexanoamido]ethyl)amine.

## Patentansprüche

1. Konjugat umfassend ein Polynucleotid, das an ein Valenzträgermolekül gekoppelt ist, wobei das Konjugat die allgemeine Formel (I) aufweist: 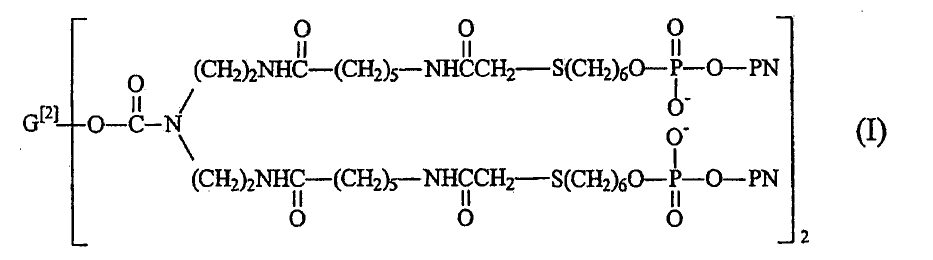 wobei G^{[2]} -CH₂(CH₂OCH₂)ᵣCH₂- ist, wobei r = 0 bis 300 und PN ein Polynucleotid ist.

2. Konjugat nach Anspruch 1, wobei G^{[2]}:
-CH₂CH₂OCH₂CH₂OCH₂CH₂-
ist.

3. Konjugat nach Anspruch 1 oder 2, wobei das Polynucleotid DNA ist.

4. Konjugat nach einem der vorstehenden Ansprüche, wobei das Polynucleotid ein Polynucleotidduplex aus mindestens 20 Basenpaaren ist.

5. Konjugat nach Anspruch 4, wobei der Duplex eine Länge von 20 bis 50 Basenpaaren aufweist.

6. Konjugat nach Anspruch 4, wobei das Polynucleotid
(CA)₁₀:(TG)₁₀
ist.

7. Konjugat nach einem der Ansprüche 4 bis 6, wobei die Polynucleotidduplexe an das Valenzträgermolekül an oder nahe einem der Enden jedes Duplexes gebunden sind.

8. Konjugat nach einem der Ansprüche 4 bis 7, wobei der Polynucleotidduplex eine wesentliche Bindungsaktivität an menschliche systemischer Lupus erythematodes-anti-dsDNA-Autoantikörper aufweist.

9. Konjugat nach Anspruch 1, das die Formel 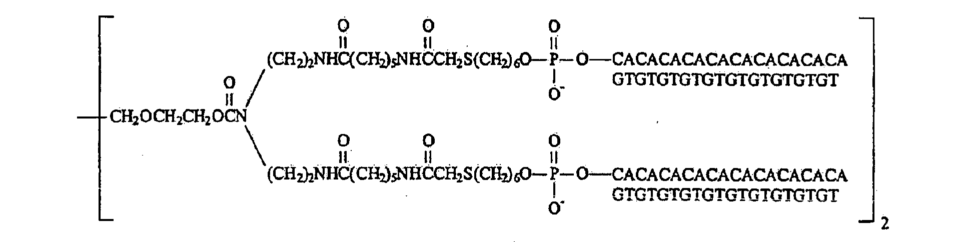 aufweist.

10. Konjugat nach Anspruch 1, wobei das Valenzträgermolekül durch Umsetzen von Polyethylenglykol-bis-chlorformiat mit N,N-Di(2-[6'-N'-carbobenzyloxyaminohexanoamido]ethyl)amin erhältlich ist.

11. Verfahren zur Herstellung eines Konjugats gemäß Anspruch 4, wobei das Verfahren umfasst:
(a) Konjugieren eines Polynucleotidlinkers der allgemeinen Formel (II): 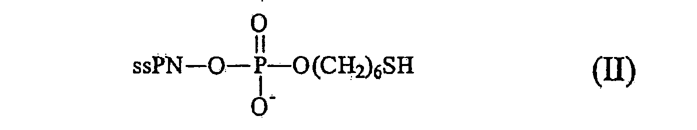 wobei ssPN ein einzelsträngiges Polynucleotid mit einer Länge von mindestens 20 Nucleotiden bedeutet, an ein Valenzträgermolekül der allgemeinen Formel (III): 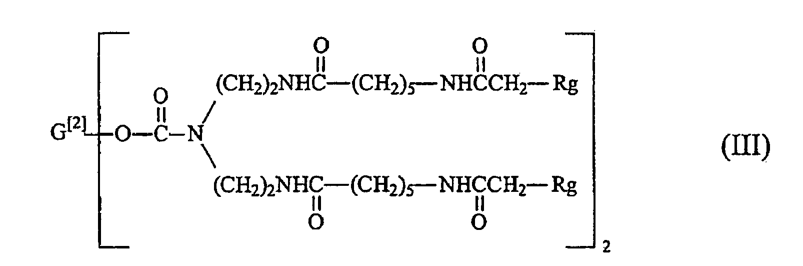 wobei Rg Halogen ist und G^{[2]} wie in Anspruch 1 definiert ist; und
(b) Anlagern von komplementären einzelsträngigen Polynucleotiden an jedes der einzelsträngigen Polynucleotide ssPN.

12. Verfahren nach Anspruch 11, wobei Rg Brom ist.

13. Verfahren nach Anspruch 11 oder 12, wobei das einzelsträngige Polynucleotid (CA)₁₀ ist und das daran angelagerte komplementäre einzelsträngige Polynucleotid (TG)₁₀ ist, so dass das Konjugat das doppelsträngige Polynucleotid: 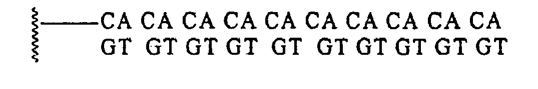 umfasst.

14. Arzneimittel, umfassend ein Konjugat gemäß einem der Ansprüche 1 bis 10 und ein pharmazeutisch verträgliches Vehikel.

15. Arzneimittel nach Anspruch 14, wobei das Vehikel ein pharmazeutisch verträglicher injizierbarer wässriger Träger ist.

16. Konjugat nach einem der Ansprüche 1 bis 10 zur Verwendung in einem Verfahren zur Behandlung des menschlichen oder tierischen Körpers durch Therapie.

17. Konjugat nach Anspruch 8 oder 9 zur Verwendung in einem Verfahren zur Behandlung von Lupus.

18. Valenzträgermolekül der allgemeinen Formel (III): 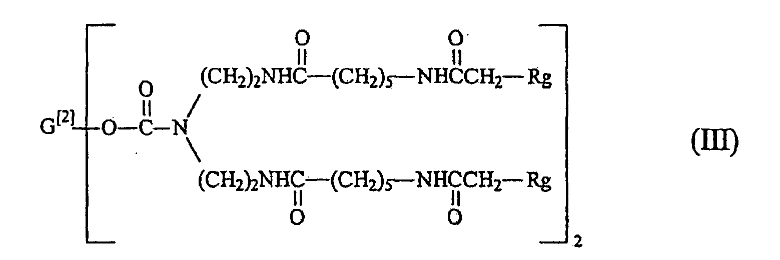 wobei G^{[2]} -CH₂(CH₂OCH₂)ᵣCH₂- ist, wobei r = 0 bis 300 und Rg Halogen ist

19. Valenzträgermolekül nach Anspruch 18, wobei G^{[2]}:
-CH₂CH₂OCH₂CH₂OCH₂CH₂-
ist.

20. Valenzträgermolekül nach Anspruch 18 oder 19, wobei Rg Brom ist.

21. Valenzträgermolekül nach Anspruch 18, das die Formel: 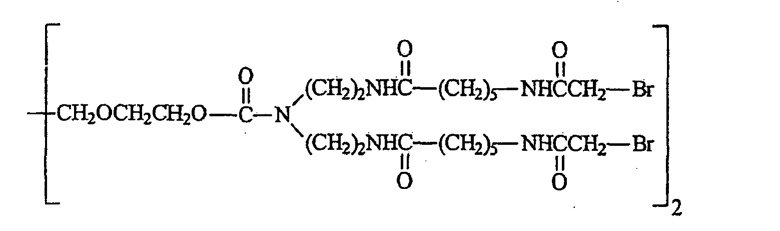 aufweist.

22. Valenzträgermolekül nach Anspruch 18, das durch Umsetzen von Polyethylenglykol-bis-chlorformiat mit N,N-Di(2-[6'-N'-carbobenzyloxyaminohexanoamido]ethyl)amin erhältlich ist.

## Revendications

1. Conjugué comprenant un polynucléotide couplé à une molécule à plate-forme de valence, lequel conjugué a la formule générale (I) : 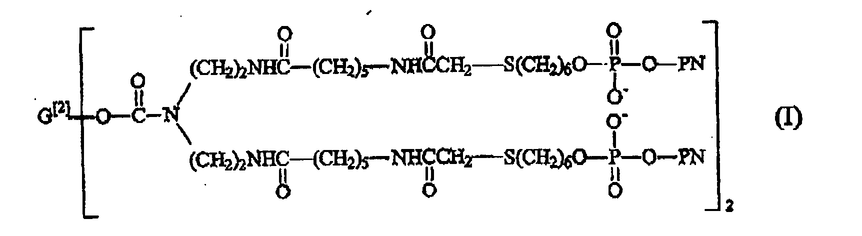 dans laquelle G^{[2]} est -CH₂(CH₂OCH₂)ᵣCH₂- où r = 0 à 300 et PN est un polynucléotide.

2. Conjugué selon la revendication 1, dans lequel G^{[2]} est :
-CH₂CH₂OCH₂CH₂OCH₂CH₂-

3. Conjugué selon la revendication 1 ou 2, dans lequel le polynucléotide est de l'ADN.

4. Conjugué selon l'une quelconque des revendications précédentes, dans lequel le polynucléotide est une double hélice polynucléotidique d'au moins 20 paires de bases.

5. Conjugué selon la revendication 4, dans lequel la double hélice est longue de 20 à 50 paires de bases.

6. Conjugué selon la revendication 4, dans lequel le polynucléotide est (CA)₁₀:(TG)₁₀.

7. Conjugué selon l'une quelconque des revendications 4 à 6, dans lequel les doubles hélices polynucléotidiques sont liées à la molécule à plate-forme de valence à ou près de l'une des extrémités de chaque double hélice.

8. Conjugué selon l'une quelconque des revendications 4 à 7, dans lequel la double hélice polynucléotidique a une activité de liaison significative pour les autoanticorps anti-ADNd de lupus érythémateux systémique humain.

9. Conjugué selon la revendication 1, qui a la formule :

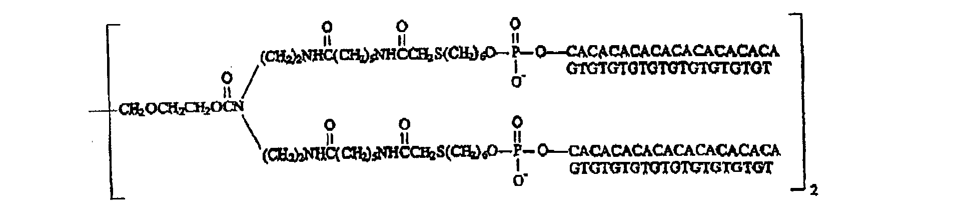

10. Conjugué selon la revendication 1, dans lequel la molécule à plate-forme de valence est susceptible d'être obtenue en faisant réagir un bis-chloroformiate de polyéthylèneglycol avec de la N,N-di(2-[6'-N'-carbobenzyloxyaminohexanoamido]éthyl)amine.

11. Procédé pour fabriquer un conjugué tel que défini dans la revendication 4, ce procédé comprenant:
(a) la conjugaison d'un lieur de polynucléotide de formule générale (II) : 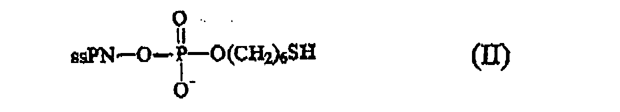 où ssPN représente un polynucléotide simple brin d'au moins 20 nucléotides de longueur, à une molécule à plate-forme de valence de formule générale (III) : 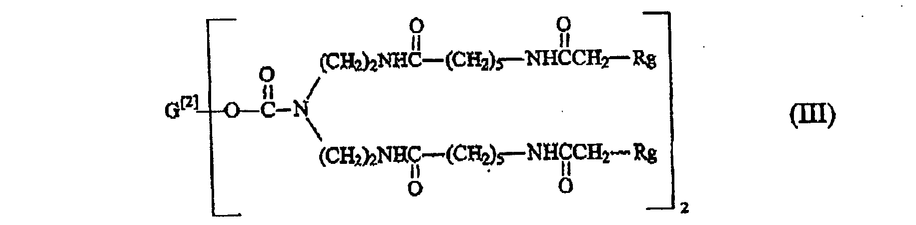 dans laquelle Rg est un halogène et G^{[2]} est tel que défini dans la revendication 1 ; et
(b) l'hybridation de polynucléotides simple brin complémentaires à chacun dudit polynucléotide simple brin ssPN.

12. Procédé selon la revendication 11, dans lequel Rg est du brome.

13. Procédé selon la revendication 11 ou 12, dans lequel le polynucléotide simple brin est (CA)₁₀ et le polynucléotide simple brin complémentaire hybridé à celui-ci est (TG)₁₀, de sorte que le conjugué comprend le polynucléotide double brin :

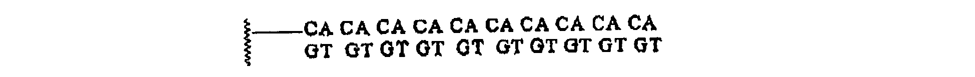

14. Composition pharmaceutique comprenant un conjugué tel que défini dans l'une quelconque des revendications 1 à 10 et un véhicule pharmaceutiquement acceptable.

15. Composition selon la revendication 14, dans laquelle le véhicule est un support aqueux injectable pharmaceutiquement acceptable.

16. Conjugué tel que défini dans l'une quelconque des revendications 1 à 10, pour l'utilisation dans un procédé de traitement du corps humain ou animal par thérapie.

17. Conjugué tel que défini dans la revendication 8 ou 9, pour l'utilisation dans un procédé de traitement du lupus.

18. Molécule à plate-forme de valence de formule générale (III) : 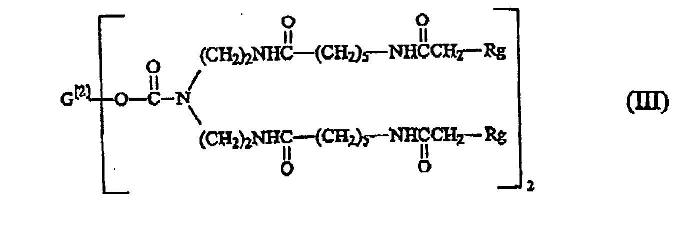 dans laquelle G^{[2]} est -CH₂(CH₂OCH₂)ᵣCH₂- où r = 0 à 300 et Rg est un halogène.

19. Molécule à plate-forme de valence selon la revendication 18, dans laquelle G^{[2]} est :
-CH₂CH₂OCH₂CH₂OCH₂CH₂-.

20. Molécule à plate-forme de valence selon la revendication 18 ou 19, dans laquelle Rg est du brome.

21. Molécule à plate-forme de valence selon la revendication 18, qui a la formule :

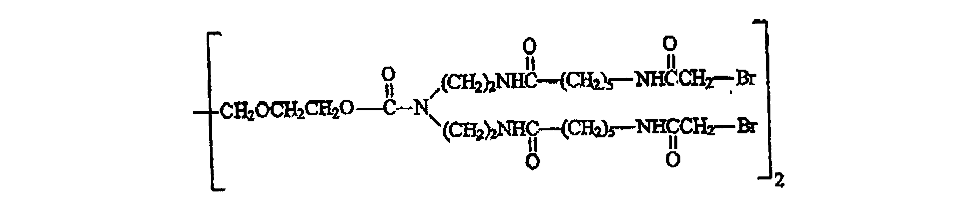

22. Molécule à plate-forme de valence selon la revendication 18, qui est susceptible d'être obtenue en faisant réagir un bis-chloroformiate de polyéthylèneglycol avec de la N,N-di(2-[6'-N'-carbobenzyloxyaminohexanoamido] éthyl)amine.
